# EUROPEAN PATENT APPLICATION

(11) **EP 4 764 495 A1**
(43) Date of publication of application: **24.06.2026**
(21) Application number: 24222895.5
(22) Date of filing: 23.12.2024
(51) Int. Cl.: G01N 33/543, B82Y 15/00, B82Y 40/00

(54) **MICROPARTICLE FOR CAPTURING ANALYTES FROM A FLUID; METHOD AND APPARATUS FOR ITS MANUFACTURE**

(71) Applicant: Technische Universität München, in Vertretung des Freistaats Bayern, 80333 München (DE)
(72) Inventor: Destgeer, Ghulam, 81827 Munich (DE); Akhtar, Muhammad Usman, 81241 München (DE); Yang, Yimin, 80939 München (DE)
(74) Representative: Lucke, Andreas

(57) **Abstract**

A microparticle for immobilizing analytes from a fluid is provided. The microparticle comprises a body. The body comprises a hydrophobic surface. The microparticle further comprises detection elements. The detection elements comprise a first detection element and a second detection element. The first detection element comprises a first hydrophilic surface. The second detection element comprises a second hydrophilic surface. The detection elements are mechanically interconnected by means of the body. The first detection element comprises, at the first hydrophilic surface, a first molecule composition for immobilizing analytes from the fluid at the first hydrophilic surface. The second detection element comprises, at the second hydrophilic surface, a second molecule composition for immobilizing analytes from the fluid at the second hydrophilic surface. The first molecule composition is different from the second molecule composition. The microparticle is non-mirror symmetric about any plane which does not intersect all of the detection elements.

## Description

### FIELD OF THE DISCLOSURE

The present disclosure relates to a microparticle for immobilizing analytes from a fluid selectively at a predefined region along the microparticle. Specifically, the present disclosure pertains to a microparticle with a hydrophobic body and hydrophilic detection elements, the hydrophilic detection elements comprising molecule compositions for immobilizing the analytes. The disclosure further relates to a method and an apparatus for fabrication of the microparticle using parallel fluid flows in a flow channel.

### BACKGROUND

For the compartmentalization of aqueous specimen, microwell plates are commonly applied. Microwell plates typically provide up to 1536 compartmentalized spaces in the form of microwells, each typically providing a volume in a range of tens of nanoliters up to several milliliters.

For high-throughput or high-resolution (i.e., in terms of analyzing smaller volumes or sub volumes of the aqueous specimen) investigations on aqueous specimen, a compartmentalization into compartments of smaller volumes and/or larger number is desirable.

### SUMMARY OF THE DISCLOSURE

It is an object of this invention to provide microparticles that overcome one or more of the disadvantages of known systems, as well as a method and an apparatus for providing such microparticles.

According to a first aspect of the disclosure, a microparticle for immobilizing analytes from a fluid is provided. The microparticle comprises a body. The body comprises a hydrophobic surface. The microparticle further comprises detection elements. The detection elements comprise a first detection element and a second detection element. The first detection element comprises a first hydrophilic surface. The second detection element comprises a second hydrophilic surface. The detection elements are mechanically interconnected by means of the body. The first detection element comprises, at the first hydrophilic surface, a first molecule composition for immobilizing analytes from the fluid at the first hydrophilic surface. The second detection element comprises, at the second hydrophilic surface, a second molecule composition for immobilizing analytes from the fluid at the second hydrophilic surface. The first molecule composition is different from the second molecule composition. The microparticle is non-mirror symmetric about any plane which does not intersect all of the detection elements.

When introduced into the fluid with the analytes, a respective microparticle provides for selective mobilization of the analytes from the fluid at the (first and second) hydrophilic surfaces of the detection elements. The selectivity is achieved by the hydrophobic/hydrophilic character of the body and the detection elements, i.e., analytes which are soluble in aqueous solutions wet the hydrophilic surfaces of the detection elements rather than the hydrophobic surface of the body. At the (first and second) hydrophilic surfaces of the detection elements, the analytes encounter the (first and second) molecule compositions, which immobilize the analytes at said surfaces.

As the molecule compositions of the detection elements, or at their hydrophilic surfaces, respectively, differ, analytes from the fluid are immobilized selectively at either of the detection elements, or at the hydrophilic surfaces thereof, respectively. Thus, the microparticle according to the invention allows for selectively immobilizing the analytes at predefined positions along the microparticle, or along its surface, respectively, so that the analytes become accessible for investigations, for example assays such as a bioassay or an immunoassay, at well-defined positions along the microparticle, for example using microscopic techniques.

The difference in molecule composition between the first and second detection elements thus allows for the simultaneous detection of multiple analytes, making the microparticle highly versatile for multiplex assays.

However, degrees of freedom which give rise to an uncertainty regarding the position of the immobilized analyte (or of the detection element that the analyte has been immobilized at, respectively) along the microparticle result from the orientational degrees of freedom of the microparticle, including the rotational degrees of freedom. In many cases, the orientational/rotational degrees of freedom of the microparticle are constrained as a microparticle typically has an anisotropic, flattened shape. When the fluid with the analytes and the microparticle are placed on a substrate defining a lower boundary of the fluid (e.g., the bottom of a well plate or a petri dish, or the surface of a microscope slide), the microparticle tends to align with its flattened side along and/or on that lower boundary. This is an immediate consequence of the flattened shape of the microparticle, because of which the potential energy of the microparticle is minimized when the microparticle is aligned with its flattened side along and/or on the lower boundary. Consequently, the remaining orientational degrees of freedom of the microparticle are the orientation around the surface normal of the lower boundary, and the binary degree of freedom as to which side of the microparticle faces up or down. Typically, a microparticle exhibits two flattened surfaces on opposite sides, and when the microparticle settles on the lower boundary of the substrate due to its high density (as compared to the density of the aqueous fluid), either of the flattened surfaces may point upwards or downwards, similar to the situation after flipping a coin.

As a consequence of the aforementioned degrees of freedom, and, in particular, for a microparticle having an overall highly symmetric shape (e.g., similar to a cylinder or a square), observing a microparticle with several detection elements from above or below (e.g., along the surface normal of said lower boundary of the substrate) typically does not allow for an unambiguous assignment of the observed detection elements to specific molecular compositions, or to immobilized analytes, respectively.

The microparticle according to the invention solves this problem by providing the microparticle with a non-mirror symmetric shape. As a consequence, the orientation of the microparticle can be determined unambiguously when the microparticle is observed from above or below (e.g., along the surface normal of said lower boundary of the substrate). This is beneficial for accurate analysis in diagnostic applications.

In the context of this disclosure, the term microparticle may refer to an object having a size of less than 1 mm along at least one spatial direction, in particular having a thickness of less than 1 mm.

Alternatively, the term microparticle may refer to an object having a size of less than 1 mm along any spatial direction.

The analytes may be biomolecules such as ligands or proteins or antigens.

The fluid may be an aqueous fluid.

Notably, the microparticle may exhibit a mirror symmetry about a mirror plane intersecting all of the detection elements and/or about a mirror plane perpendicular to a thickness direction of the microparticle.

The thickness direction may correspond to a direction, along which the spatial extension of the microparticle is the smallest.

The thickness direction may be a direction from the top surface to the bottom surface.

According to an embodiment, the body comprises a marker structure. The marker structure is non-mirror symmetric about any plane which does not intersect all of the detection elements.

The inclusion of a marker structure that is non-mirror symmetric enhances the microparticle's ability for its orientation to be identified during analysis. This asymmetry in the marker structure aids in distinguishing the orientation of the microparticle and ensures a correct assignment of the individual detection elements.

In particular, since the marker structure is part of the body, it can be observed in bright-field microscopy, for example in reflected light microscopy or in transmitted light microscopy. In other words, a respective marker structure facilitates a quick detection of the orientation of the microparticle using well-established and simple techniques.

According to some embodiments, the marker structure is associated with an outer surface of the body. In particular, the marker structure may be formed by the outer surface of the body or may be arranged on the outer surface of the body.

Optionally, the body has a top surface and a bottom surface opposite to the top surface, and at least part of the marker structure is associated with the top surface and/or with the bottom surface. In particular, the at least part of the marker structure may be associated with a circumferential shape of the top surface and/or with a circumferential shape of the bottom surface. In particular, the marker structure may comprise recesses in the circumferential shape of the top surface and/or in the circumferential shape of the bottom surface. In particular, at least two of said recesses may have different shapes and/or at least two of said recesses may be comprised in the same one of the circumferential shape of the top surface and the circumferential shape of the bottom surface. In particular, said at least two of the recesses may have different shapes and may both be comprised in both the circumferential shape of the top surface and the circumferential shape of the bottom surface.

The marker structure being associated with the outer surface of the body allows for easy identification and differentiation of the microparticle, in particular under a microscope and using brightfield microscopy. This can be particularly useful in applications where multiple microparticles are used, as it enables quick visual or automated recognition.

According to an embodiment, the microparticle comprises at least two different marker materials having different optical properties, wherein the at least two different marker materials are arranged in the microparticle non-mirror symmetrically about any plane which does not intersect all of the detection elements.

Similar to the marker structure, the inclusion of at least two different marker materials with different optical properties allows for detecting the orientation of the microparticle. The non-mirror symmetrical arrangement ensures that the detection elements can be uniquely identified based on their optical signatures, enhancing the accuracy and reliability of analyte detection.

The different marker materials may be applied as an alternative to the marker structure, or they may be applied additionally, to make the orientation accessible to a larger variety of detection techniques, including photoluminescence microscopy.

In respective embodiments, the first detection element may comprise a first marker material of the at least two different marker materials at a higher concentration, in particular in terms of weight percent or in terms of molar concentration, than the second detection element, and the second detection element may comprise a second marker material of the at least two different marker materials at a higher concentration, in particular in terms of weight percent or in terms of molar concentration, than the first detection element. In particular, said higher concentrations may be higher at least by a factor of two or at least by a factor of five or at least by a factor of ten.

In respective embodiments, the first detection element may comprise a first marker material of the at least two different marker materials at a higher concentration, in particular in terms of weight percent or in terms of molar concentration, than the body. In particular, this higher concentration may be higher at least by a factor of two or at least by a factor of five or at least by a factor of ten.

Alternatively, or in addition, the second detection element may comprise a second marker material of the at least two different marker materials at a higher concentration, in particular in terms of weight percent or in terms of molar concentration, than the body. In particular, this higher concentration may be higher at least by a factor of two or at least by a factor of five or at least by a factor of ten.

The different optical properties may comprise at least one of the following: different photoluminescence emission wavelengths, and maxima at different wavelengths in corresponding optical absorption spectra.

Alternatively, or in addition, the at least two different marker materials may comprise or may be at least two different photoluminescent dyes. In particular, the at least two different marker materials may comprise or may be at least two different fluorescent dyes.

Using different photoluminescent or fluorescent dyes as marker materials enhances the optical detection capabilities of the microparticle. These dyes provide strong and distinct optical signals, which improve the sensitivity in determining the orientation of the microparticle.

According to an embodiment, the analytes comprise analytes of a first type, and the first molecule composition comprises immobilization molecules of a first type at a higher concentration than the second molecule composition. In respective embodiments, the immobilization molecules of the first type may be adapted to immobilize analytes of the first type at the respective surface comprising the respective molecule composition comprising the immobilization molecules of the first type.

Respective embodiments beneficially ensure that analytes of the first type get immobilized at the first hydrophilic surface, providing them at a predefined position along the microparticle for further analysis.

In respective embodiments, the immobilization molecules of the first type may be adapted to selectively immobilize the analytes of the first type at the respective surface comprising the respective molecule composition comprising the immobilization molecules of the first type.

The higher concentration may refer to a higher concentration in terms of weight percent or in terms of molar concentration.

The higher concentration may be higher at least by a factor of two, or at least by a factor of five, or at least by a factor of ten.

The immobilization molecules of the first type may be essentially absent in the second molecule composition.

According to an embodiment, the analytes comprise analytes of a first type and analytes of a second type. In respective embodiments, the first molecule composition may comprise immobilization molecules of a first type for selectively immobilizing the analytes of the first type from the fluid at the first hydrophilic surface, and the second molecule composition may comprise immobilization molecules of a second type for selectively immobilizing the analytes of the second type from the fluid at the second hydrophilic surface.

A respective microparticle can effectively differentiate and capture multiple types of analytes from a fluid. This selective immobilization enhances the specificity and efficiency of the microparticle in capturing target analytes, providing a significant technical improvement in applications such as diagnostics and assays such as biochemical assays.

The microparticle for immobilizing the analytes from the fluid may refer to a microparticle for immobilizing analytes of the first type and of the second type different from the first type from the fluid, in particular, selectively at different surface regions along the microparticle.

The first molecule composition may comprise the immobilization molecules of the first type at a higher concentration, in particular in terms of weight percent or in terms of molar concentration, than the second molecule composition and optionally than the body, in particular, at least by a factor of two or at least by a factor of five or at least by a factor of ten.

The second molecule composition may comprise the immobilization molecules of the second type at a higher concentration, in particular in terms of weight percent or in terms of molar concentration, than the first molecule composition and optionally than the body, in particular, at least by a factor of two or at least by a factor of five or at least by a factor of ten.

According to an embodiment, the first detection element comprises a first cavity, and at least part of the first hydrophilic surface is a surface of the first cavity, and the second detection element comprises a second cavity, and at least part of the second hydrophilic surface is a surface of the second cavity.

The presence of cavities in the detection elements allows for an increased surface area for analyte immobilization, enhancing the immobilization capability of the microparticle. In particular, as an analyte having diffused into one of the cavities is surrounded by the corresponding hydrophilic surface on multiple sides, the probability that the analyte gets immobilized is improved (i.e., enhanced) significantly.

Further, the first and second hydrophilic surfaces interact with an aqueous fluid that the microparticle is placed in. The aqueous fluid wets the respective surfaces. The aqueous fluid having wetted the first/second hydrophilic surfaces is sealed by the body and/or by the first/second detection element. Preferably, the microparticle is applied in a fluid comprising an oil, more specifically, and/or is transferred to a fluid comprising an oil after exposure to the fluid comprising the analyte. The oil seals the cavities, and thus the fluid having wetted the first/second hydrophilic surfaces. This results in sealed nano-liter compartments within microparticles.

The first cavity and the second cavity may be separated from one another by means of the body.

Separating the cavities by the body ensures that there is no cross-contamination between the different molecule compositions on the first and second hydrophilic surfaces. This separation maintains the integrity of the detection process for each type of analyte, leading to more accurate and reliable results.

The compartments within the microparticle and separated by the body also allow for signal accumulation for different types of analytes within corresponding cavities of the microparticle, while minimizing cross-contamination at the same time.

Said surface of the first cavity may refer to an inner surface of the first cavity and said surface of the second cavity may refer to an inner surface of the second cavity.

A respective geometry enhances the probability for successful immobilization further.

The first cavity and/or the second cavity may be formed as a through hole extending from a bottom surface of the microparticle to a top surface of the microparticle, the top surface and the bottom surface being opposite surfaces of the microparticle.

A respective geometry enhances the probability for successful immobilization further.

In addition, the through-hole geometry allows for subsequent analysis of the analyte immobilized on the respective hydrophilic surface using transmission microscopy, as light can be shone through the through hole.

According to some embodiments, the detection elements may be embedded in the body, in particular, the first cavity and the second cavity may be embedded in the body.

According to an embodiment, the microparticle has a thickness of less than 1 mm, in particular from a top surface of the microparticle to a bottom surface of the microparticle, the bottom surface being located opposite to the top surface, in particular, wherein the top surface and the bottom surface are parallel to each other and/or wherein an aspect ratio between the thickness of the microparticle and a width of the top surface and/or of the bottom surface is no more than 1:1 or no more than 1:2 or no more than 1:2.5 or no more than 1:3.

A respective geometry beneficially ensures that the microparticle aligns on a substrate (e.g., at the bottom of the well plate or on a microscope slide) with one of the top surface and the bottom surface pointing towards the substrate, and the other one of the top surface and the bottom surface pointing in the opposite direction, which is typically the opposite direction with respect to an external reference frame defined by the local direction of the gravitational field of the earth. Thus, the rotational degrees of freedom of the microparticle get constrained from three rotational degrees of freedom to a single rotational degree of freedom plus the binary degree of freedom of either the top surface or the bottom surface pointing upwards along the local direction of the gravitational field of the earth.

The microparticle may have a width of less than 3 mm, in particular of less than 1 mm and/or along any spatial direction.

A microparticle with a respective width can beneficially be fabricated using the stop flow lithography method described below.

Moreover, a microparticle with the respective width allows for compartmentalization into detection volumes, as defined by the detection elements, on the nanoliter scale.

The microparticle may be shaped to permit, when the microparticle is placed in the fluid, the fluid to reach the first hydrophilic surface and to reach the second hydrophilic surface.

The body may comprise or may consist of a body material, the body material being hydrophobic. The body material may be a polymer.

The hydrophobic body material ensures a hydrophobic surface of the body.

A hydrophobic surface of the body prevents non-specific binding of analytes to the body, directing them instead to the hydrophilic detection elements. This selective interaction improves the accuracy of analyte detection by reducing background noise and enhancing signal clarity.

The body having the hydrophobic surface ensures a separation between the detection elements, thus ensuring that the detection elements can be resolved individually, e.g., in a microscopic measurement.

The body having the hydrophobic surface also ensures a separation between the detection elements and the outer circumference of the microparticle, thus ensuring that detection elements of different microparticles can be resolved individually, even if the microparticles are in contact with each other, e.g., in a microscopic measurement.

The body having the hydrophobic surface also assists, when the aqueous fluid comprising the analyte has wetted the hydrophilic surfaces of the detection elements, and when the microparticle is exposed to the fluid comprising oil, the oil of the fluid in sealing the aqueous fluid. This sealing beneficially supports signal accumulation. The inventors have found that, after sealing, the signal accumulation increases with time, providing an even further amplified signal, e.g., for a diagnostic immunoassay.

The body having the hydrophobic surface thus aids in signal accumulation, allowing to increase the signal over time, which can be then clearly distinguished from a reference background. The body having the hydrophobic surface therefore gives rise to an improved contrast between the signal and the background.

Microparticle, and in particular, the combination of the detection elements with the body having the hydrophobic surface, thus improve the accuracy and sensitivity of an assay performed with the microparticle.

The improved accuracy can be attributed to better statistical confidence, in particular, when multiple, e.g., hundreds of microparticles are applied simultaneously.

The increased signal improves the detection limit of the assay by providing a quantifiable signal, even from a small amount of analytes, or in case of a fluid comprising the analyte at very low concentration, respectively, for example, at a picomolar or femtomolar concentration. The microparticle thus provides an improved detection of analytes.

The first detection element may comprise or may consist of a first material, the first material being hydrophilic. The first material may be a polymer.

The second detection element may comprise or may consist of a second material, the second material being hydrophilic, in particular, wherein the second material is the same as the first material. The second material may be a polymer.

The hydrophilic first material of the first/second detection element ensures a (first/second) hydrophilic surface of the first/second detection element.

The hydrophilic surface of the first/second detection element promotes strong interactions with analytes in the fluid, facilitating their immobilization on the surface. This characteristic enhances the efficiency and effectiveness of the immobilization process and of a further analysis and detection process.

Forming at least part of the first and second detection element from the same first/second material ensures efficient fabrication of the detection elements.

The first detection element and the second detection element may be encircled by a top surface of the microparticle, in particular, wherein the first detection element and the second detection element are encircled by the top surface of the microparticle and by a bottom surface of the microparticle, the top surface and the bottom surface being opposite surfaces of the microparticle.

The first detection element and the second detection element may be encircled by a circumferential shape of the top surface of the microparticle and/or the first detection element and the second detection element may be encircled by a circumferential shape of the top surface of the microparticle and by a circumferential shape of the bottom surface of the microparticle. In particular, the circumferential shape of the bottom surface of the microparticle and/or the circumferential shape of the top surface of the microparticle may be defined by the body of the microparticle.The encircling may refer to or may be an encircling in a plane defined by the bottom surface or defined by the bottom surface.

Encircling the detection elements with the top and bottom surfaces provides structural integrity and protection, ensuring that the detection elements remain intact and functional during use.

The detection elements may further comprise a third detection element comprising a third hydrophilic surface, wherein the third detection element at the third hydrophilic surface comprises a third molecule composition for immobilizing analytes from the fluid at the third hydrophilic surface, wherein the third molecule composition is different from the first molecule composition and from the second molecule composition.

Including a third detection element with a distinct molecule composition allows for the simultaneous detection of multiple analytes within the same microparticle. This feature increases the versatility and application range of the microparticle, enabling it to be used in complex analytical scenarios.

The detection elements may further comprise a fourth detection element comprising a fourth hydrophilic surface, wherein the fourth detection element at the fourth hydrophilic surface comprises a fourth molecule composition for immobilizing analytes from the fluid at the fourth hydrophilic surface, wherein the fourth molecule composition is different from the first molecule composition, from the second molecule composition and from the third molecule composition.

The addition of a fourth detection element further expands the microparticle's capability to detect a wider range of analytes simultaneously. This feature enhances the microparticle's utility in comprehensive analytical applications, providing a robust tool for multi-analyte detection.

The detection elements may be separated from one another by means of the body.

Separation of detection elements by the body prevents cross-contamination and interference between different analyte interactions. This spatial separation ensures that each detection element functions independently, improving the specificity and accuracy of the detection process.

Moreover, a separation between the detection elements facilitates reliable, separate detection of optical signals selectively from one of the detection elements.

According to a second aspect of the disclosure, a plurality of microparticles is provided. Each microparticle of the plurality comprises the marker structure according to any of the embodiments described above, and/or each microparticle comprises the at least two different marker materials according to any of the embodiments described above.

The arrangement of the marker structure and/or marker materials is consistent across the microparticles in the plurality. This uniformity ensures that the microparticles behave predictably and produce reliable results in assays. It also simplifies the manufacturing process, as each microparticle is produced to the same specifications, reducing variability and increasing the reproducibility of results.

In preferred embodiments, the arrangement of the marker structure and/or of the marker materials may be the same in the microparticles of the plurality of microparticles.

In some embodiments, a kit is provided, comprising a first plurality of microparticles and a second plurality of microparticles. Each microparticle of the first plurality comprises a first marker structure, the first marker structure being a marker structure according to any of the embodiments described above, and/or each microparticle of the first plurality comprises a first set of different marker materials, the first set of different marker materials comprising the at least two different marker materials according to any of the embodiments described above. Each microparticle of the second plurality comprises a second marker structure, the second marker structure being a marker structure according to any of the embodiments described above, and/or each microparticle of the second plurality comprises a second set of different marker materials, the second set of different marker materials comprising the at least two different marker materials according to any of the embodiments described above. In respective embodiments, the first set of different marker materials may be different from the second set of different marker materials. Alternatively, or in addition, the first marker structure may be different from the second marker structure.

Respective embodiments are particularly beneficial if, for example, a first vial V1 has the first plurality of microparticles with a first marker structure, lets call it P1. A second vial V2 has the second plurality of microparticles with similar detection elements to the detection elements of the microparticles of the first plurality, but with different marker structure, P2. A biosample coming from patient 1 having a specific analyte concentration is mixed with P1 in vial V1, and a biosample from patient 2 is mixed with P2 in vial V2. After performing the first steps of an assay in separate vials V1 and V2, P1 will bind analyte concentration corresponding to patient 1 and P2 will bind analyte concentration specific to patient 2. Then P1 and P2 can be mixed and the remaining steps of the assay can be performed together. This will save effort, materials, and time. Upon imaging within one detection element containing two populations of particles P1 and P2 that can be distinct based on marker arrangement, the signal from one detection element demonstrates the concentration of analytes for two patients.

The plurality of microparticles may comprise at least five or at least 10 or at least 100 microparticles.

According to a third aspect of the disclosure, a method is provided for fabricating the microparticle according to any of the embodiments above. The method comprises providing parallel flows of fluids in a flow channel. The parallel flows of fluids comprise a flow of a fluid comprising a precursor for a hydrophobic material. The parallel flows of fluids further comprise a flow of a first additional fluid and a flow of a second additional fluid. The first additional fluid comprises a precursor for a hydrophilic material, and the second additional fluid comprises a precursor for a hydrophilic material. The method further comprises curing the precursor for the hydrophobic material into a hydrophobic material forming at least part of the body, and curing the precursor for the hydrophilic material comprised in the first additional fluid into a hydrophilic material forming at least part of the first detection element. Additionally, the method comprises curing the precursor for the hydrophilic material comprised in the second additional fluid into a hydrophilic material forming at least part of the second detection element.

Optionally, the provided parallel flows of the fluids comprise a common solvent.

Any of the curing processes described above may comprise a photochemical reaction, and/or may refer to a curing using illumination, in particular illumination with UV radiation.

Any of the said hydrophilic materials may be a hydrophilic polymer, and/or the hydrophobic material may be a hydrophobic polymer.

The term flow channel may refer to an object adapted to permit a fluid flow through the flow channel along a flow direction defined by the flow channel, the flow channel comprising sidewalls that enclose the fluid flow in a fluid-tight manner in the plane perpendicular to the flow direction.

The first additional fluid and the second additional fluid may comprise immobilization molecules, such that the first molecule composition comprises immobilization molecules from the first additional fluid and the second molecule composition comprises immobilization molecules from the second additional fluid. In particular, the immobilization molecules comprised in the first additional fluid may comprise immobilization molecules of a first type and the immobilization molecules comprised in the second additional fluid may comprise immobilization molecules of a second type.

Providing parallel flows of fluids in a flow channel ensures that the different precursor materials can be accurately controlled and directed. This allows for precise fabrication of the microparticle's structure, ensuring that each material is correctly positioned.

The flow of a first/second additional fluid comprising a precursor for a hydrophilic material allows for the formation of a (first/second) detection element with a (first/second) hydrophilic surface. This hydrophilic layer can be functionalized to interact with specific biomolecules, making it useful for applications such as biomarker detection. Having multiple detection elements, or multiple hydrophilic surfaces, respectively allows the microparticle to simultaneously detect multiple biomarkers, enhancing its diagnostic capabilities.

Curing the precursors into hydrophobic/hydrophilic material ensures that the microparticle is solidified and stable, providing the structural integrity of the microparticle, and well-defined positions of the detection elements along the fabricated microparticle.

The common solvent improves the parallelicity of the flows and thus the reliability and resolution of the fabrication method.

The common solvent allows hydrophobic and hydrophilic material to coflow in parallel streamlines without phase separation. The common solvent makes them partially miscible. The hydrophobic and hydrophilic material flowing parallel to each other can diffuse to some extent at the interface. This improves the quality and the resolution of the fabricated microparticles.

In the context of this disclosure, the term precursor may refer to a precursor for polymerization, or to a precursor for forming a polymer therefrom, respectively. In particular, the term precursor may refer to a photochemical precursor suitable for polymerization under illumination, such as illumination with UV light.

According to a further aspect of the disclosure, an apparatus is provided for fabricating the microparticle according to any of the embodiments described above. The apparatus comprises a flow channel. The flow channel comprises at least three inlet openings for providing parallel flows of fluids in the flow channel. Each of the at least three inlet openings is adapted to provide a respective one of the parallel flows of the fluids in the flow channel. The flow channel further comprises a reducing section located downstream from the at least three inlet openings. A cross-sectional area of the flow channel at a downstream end of the reducing section is smaller than a cross-sectional area of the flow channel at an upstream end of the reducing section. The flow channel further comprises tapered elements arranged in the flow channel and at least partially in the reducing section of the flow channel. Each of the tapered elements has a smaller cross-sectional area at a downstream end of the respective tapered element than at an upstream end of the respective tapered element. The at least three inlet openings comprise a first inlet opening located in the flow channel at a first lateral position and a second inlet opening located in the flow channel at a second lateral position laterally offset from the first lateral position. The tapered elements comprise a first tapered element arranged downstream from the first inlet opening at a lateral position corresponding to the first lateral position, to reduce a cross-sectional area of the one of the parallel flows of the fluids provided by the first inlet opening. The tapered elements further comprise a second tapered element arranged downstream from the second inlet opening at a lateral position corresponding to the second lateral position, to reduce a cross-sectional area of the one of the parallel flows of the fluids provided by the second inlet opening.

According to an embodiment, the flow channel comprises at least four inlet openings for providing the parallel flows of fluids in the flow channel.

According to an embodiment, the flow channel comprises at least five inlet openings for providing the parallel flows of fluids in the flow channel.

According to an embodiment, the flow channel comprises at least six inlet openings for providing the parallel flows of fluids in the flow channel.

According to an embodiment, the flow channel comprises at least seven inlet openings for providing the parallel flows of fluids in the flow channel.

The one of the parallel flows of the fluids provided by the first inlet opening may refer to the flow of the first additional fluid described above in the context of the method.

The one of the parallel flows of the fluids provided by the second inlet opening may refer to the flow of the second additional fluid described above in the context of the method.

The apparatus comprises a flow channel with at least three inlet openings, enabling parallel flows of fluids. This configuration allows for the simultaneous introduction of multiple fluid streams, which is beneficial for creating complex multi-layered particles, with the advantages described above in the context of the microparticle.

Tapered elements within the flow channel, particularly in the reducing section, further refine the flow by reducing the cross-sectional area of each stream. This design enhances the precision of particle formation, since the flows can be sculpted with a larger cross-section, and then their cross sections can be "compressed" into the one of the desired microparticle. This approach allows for the creation of particles with complex geometries at high resolution, and thus for the production of the microparticle described above in the context of the first aspect.

The arrangement of the first and second inlet openings at laterally offset positions, along with corresponding tapered elements, allows for the independent manipulation of fluid streams. This spatial configuration is beneficial for creating particles with distinct regions or compartments, each potentially carrying different functional materials, thereby increasing the versatility and functionality of the particles.

Notably, in the context of this disclosure, the terms "downstream" and "upstream" do not require an actual flow of a fluid through the channel to be well-defined. To the contrary, the terms "downstream" and "upstream" are to be understood relative to each other, i.e., they indicate opposing directions along the flow channel, and are defined this way also in the absence of the flow of a fluid through the channel. In other words, the terms "downstream" and "upstream" are used for illustrative purposes and could alternatively be replaced by the terms "first direction" and "second direction opposite to the first direction". If the terms were to be replaced accordingly, the reducing section may serve as a reference, i.e., the cross-sectional area of the flow channel at the end of the reducing section in the first direction would be smaller than a cross-sectional area of the flow channel at the end of the reducing section in the second direction opposite to the first direction, and the reducing section would be located along the first direction after/behind the at least three inlet openings.

Each of the tapered elements may extend along a direction parallel to a flow direction defined by the flow channel or may enclose a line parallel to a flow direction defined by the flow channel.

According to an embodiment, the tapered elements further comprise an outer tapered element around the first tapered element and the second tapered element.

The outer tapered element works in conjunction with the first and second tapered elements to further refine the flow of fluids within the flow channel. This additional tapered element provides an extra level of control over the fluid dynamics, ensuring that the parallel flows are more precisely directed and reduced in cross-sectional area, leading to an improved resolution and reliability of the method and of the microparticles being fabricated.

The outer tapered element may surround the first tapered element and the second tapered element in a plane perpendicular to a flow direction defined by the flow channel.

The outer tapered element may have an asymmetric shape around a centerline of the flow channel, in particular, wherein the asymmetric shape refers to a shape without a rotational symmetry around the center line, in particular, to a shape without a discrete or continuous rotational symmetry around the centerline.

The outer tapered element may comprise at least one notch, in particular, at least two notches at different lateral positions along the outer tapered element.

The at least two notches at the different lateral positions along the outer tapered element may have different sizes and/or the at least two notches may further comprise a third notch (or may be at least three notches, respectively).

The symmetrical shape of the outer tapered element or the notch(es) beneficially break any mirror symmetry of the fabricated microparticle about any plane which does not intersect all of the detection elements, with the advantages described above in the context of the microparticle according to the first aspect of this disclosure.

According to an embodiment, the at least three inlet openings comprise at least one outer inlet opening. In respective embodiments, according to a projection onto a plane perpendicular to a flow direction defined by the flow channel, each of the at least one outer inlet opening may be arranged around the first inlet opening and around the second inlet opening.

The outer inlet opening(s) beneficially provide further streams of fluids, in particular of the fluid, which is cured to form the body, and/or a stream of a carrier fluid surrounding the other parallel streams of fluids and having a concentration of precursors lower than any of the other streams of the fluids.

In respective embodiments, the at least one outer inlet opening may comprise a first outer inlet opening. In respective embodiments, the outer tapered element may be arranged downstream from the first outer inlet opening and, according to the projection onto the plane perpendicular to the flow direction defined by the flow channel: the upstream end of the outer tapered element may be arranged around the first outer inlet opening and/or the outer tapered element may overlap the first outer inlet opening.

A respective first outer inlet opening beneficially provides a stream of a fluid suitable for forming the body therefrom. Positioning the outer tapered element downstream from the first outer inlet opening and arranging it around or overlapping the first outer inlet opening ensures that the fluid flow from the outer inlet is effectively guided and constricted by the tapered element, resulting in a more focused and controlled flow, improving the precision at which the shape of the body is provided.

The first outer inlet opening may be adapted to provide the flow of the fluid comprising the precursor for the hydrophobic material as described above in the context of the method.

The flow of the fluid comprising the precursor for the hydrophobic material, as described above in the context of the method, may be provided by the first outer inlet opening.

The at least one outer inlet opening may comprise a second outer inlet opening.

The outer tapered element may be arranged downstream from the second outer inlet opening and, according to the projection onto the plane perpendicular to the flow direction defined by the flow channel: the second outer inlet opening may be arranged around the downstream end of the outer tapered element and/or the outer tapered element may overlap the second outer inlet opening.

A respective second outlet opening beneficially provides an additional stream of a fluid, namely of a carrier fluid which avoids that the material of the body and/or of the detection elements sticks to the walls of the channel and clogs the channel when cured.

According to an embodiment, the apparatus further comprises a lithographic mask arranged in a lithographic masking section of the apparatus, wherein a section of the flow channel passes through said lithographic masking section, and wherein the lithographic mask is arranged in the lithographic masking section such that, under illumination of the mask with a UV radiation having a direction towards said section of the flow channel, said UV radiation is masked with the lithographic mask before reaching said section of the flow channel.

The masking enables precise control over the exposure of the fluids within the flow channel to UV radiation, thereby allowing for accurate patterning and structuring of the microparticles being fabricated.

The lithographic mask may comprise slits having segments with an extension perpendicular to a flow direction defined by the flow channel in the lithographic masking section, said slits being adapted to allow said UV radiation to reach said section of the channel, wherein the lithographic mask is adapted to block at least a part of the UV radiation or the UV radiation not passing through the slits.

The apparatus may further comprise a UV source adapted to provide said UV radiation having the direction towards said section of the flow channel.

According to a further aspect of the disclosure, the method described above in the context of the third aspect of this disclosure applies the apparatus according to any of the embodiments described above; wherein the parallel flows of the fluids in the flow channel are provided via the at least three inlet openings; wherein the flow of the first additional fluid is provided from the first inlet opening; and wherein the flow of the second additional fluid is provided from the second inlet opening.

In respective embodiments, the flow of the fluid comprising the precursor for the hydrophobic material may be provided from one of the at least one outer inlet opening described above, in particular, may be provided from the first outer inlet opening.

The parallel flows of the fluids provided in the flow channel may further comprise a carrier flow, in particular, wherein the carrier flow may be provided from one of the at least one outer inlet opening described above, in particular, may be provided from the second outer inlet opening described above.

In the context of this disclosure, a carrier flow may refer to one of the parallel flows of the fluids, the carrier flow being a flow of a fluid comprising a lower concentration of the precursor for the hydrophobic material than the flow of the fluid comprising the precursor for the hydrophobic material; and comprising a lower concentration of the precursor for the hydrophilic material than the flow of the first additional fluid; and comprising a lower concentration of the precursor for the hydrophilic material than the flow of the second additional fluid. In particular, said higher concentrations may refer to higher concentrations in terms of weight percent or in terms of molar concentration and/or said higher concentrations may be higher at least by a factor of two or at least by a factor of five or at least by a factor of ten.

### LIST OF FIGURES

In the following, a detailed description of the present disclosure and examples thereof is given with reference to the figures, wherein
Fig. 1a and Fig. 1b give a schematic illustration of a microparticle according to an example of the present disclosure;
Fig. 2a and Fig. 2b, Fig. 3a and Fig. 3b, Fig. 4a and Fig. 4b, Fig. 5a and Fig. 5b, Fig. 6a and Fig. 6b, Fig. 7a and Fig. 7b, Fig. 8a and Fig. 8b each give schematic illustrations of a microparticle according to a further example of the present disclosure;
Fig. 9 schematically illustrates a method according to an example of the present disclosure;
Fig. 10a to Fig. 10n schematically illustrate an apparatus according to an example of the present disclosure;
Fig. 11 schematically illustrates an apparatus according to a further example of the present disclosure;
Fig. 12 and Fig. 13 schematically illustrate lithographic masking sections of apparatus according to different examples of the present disclosure;
Fig. 14 schematically illustrates a lithographic mask of an apparatus according to an example of the present disclosure;
Fig. 15 schematically illustrates a lithographic masking section and a reducing section of a flow channel, of an apparatus according to an example of the present disclosure;
Fig. 16 and Fig. 17 schematically illustrate the parallel flows of fluids inside the flow channel in different planes perpendicular to the flow direction;
Fig. 18 and Fig. 19 schematically illustrate outer tapered elements of apparatus according to different examples of the present disclosure;
Fig. 20 schematically illustrates the parallel flows of fluids inside the flow channel in a plane perpendicular to the flow direction, in apparatus having outer tapered elements with different shapes;
Fig. 21 gives a photograph of microparticles according to an example of the present disclosure;
Fig. 22 schematically illustrates an apparatus according to a further example of the present disclosure;
Fig. 23 schematically illustrates an apparatus according to a further example of the present disclosure;
Fig. 24 schematically illustrates a method of using a microparticle according to an example of the present disclosure;
Fig. 25 schematically illustrates another method of using a microparticle according to an example of the present disclosure; and
Fig. 26a and Fig. 26b schematically illustrate results of the method of Fig. 24.

### DESCRIPTION OF EXAMPLES

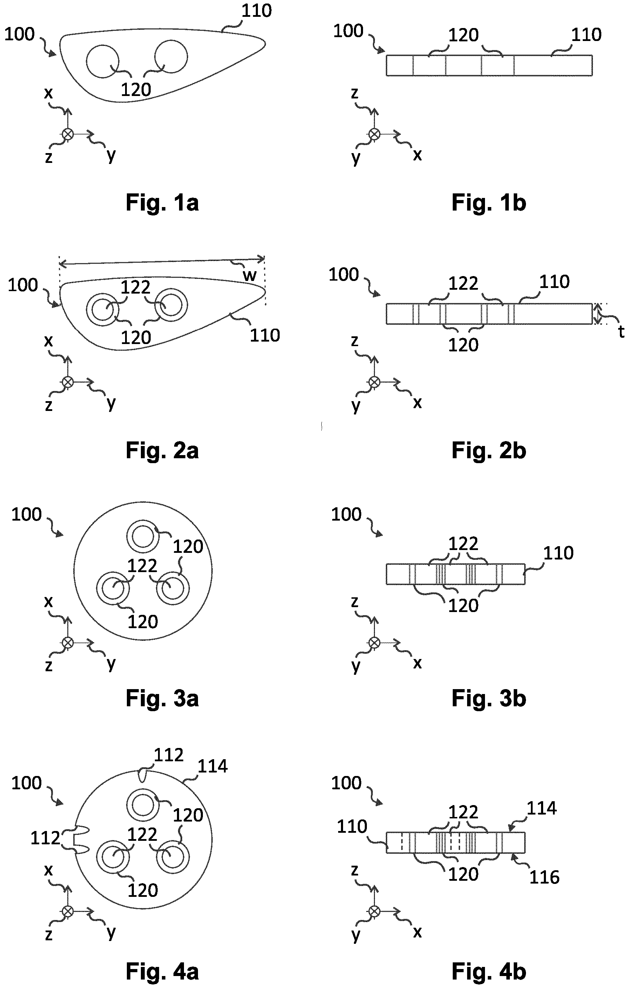

Fig. 1a and Fig. 1b illustrate a microparticle 100, comprising a body 110 and detection elements 120.

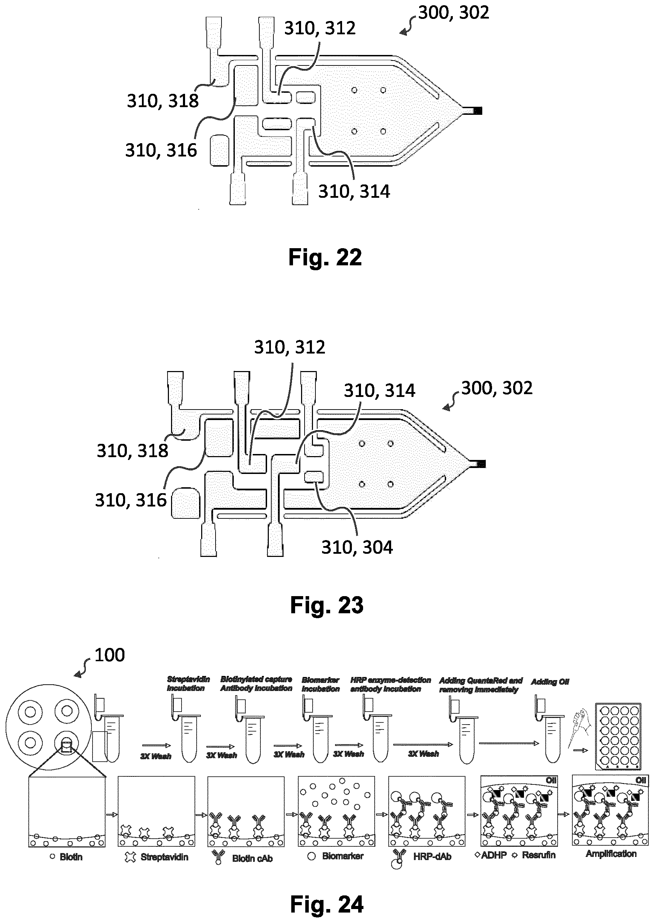

The microparticle 100 can beneficially be applied in an assay, for example in a bioassay or in an immunoassay, as will be described in detail below in the context of Fig. 24 to Fig. 26b. Generally speaking, for this purpose, the microparticle 100 is placed in a fluid arranged over/above a substrate, such as a well of a microplate, a petri dish or a suitable microscope slide. Analytes from the fluid are immobilized selectively at the detection elements 120, or at hydrophilic surfaces thereof, respectively. Consequently, the concentration of the analytes is drastically increased at the detection elements 120, or at their hydrophilic surfaces, respectively, as compared to what the concentration of the analytes would be in the fluid in the absence of the microparticle 100. The increased concentration of the analytes at the detection elements 120 facilitates measurements and analysis thereon with increased signal strength and thus with an increased accuracy, in particular, when a spatially resolved measurement and analysis technique is used, for example making use of a microscope. In other words, the microparticle 100 facilitates a high reliability measurement/analysis even on analytes comprised in the fluid in small amounts or at small concentration. This concerns, on the one hand, fluids with a large overall volume but a small concentration of the analyte, as the microparticle 100 can immobilize, capture and concentrate the analyte spatially distributed in the fluid. On the other hand, signals from small amounts of analytes, resulting from a small overall amount of the liquid they are comprised in, can be amplified and the microparticle 100 thus permits measurements on fluid volumes on the nanoliter scale.

In the depicted embodiment, the body 110 is formed from a hydrophobic polymer without any further coating, thus providing a hydrophobic surface of the body 110.

In the depicted embodiment, the detection elements 120 each comprise a hydrophilic polymer. The detection elements 120 are formed without any further coating, such that the hydrophilic polymers provide hydrophilic surfaces of the detection elements 120.

The body's 110 hydrophobic nature minimizes non-specific binding at/to the body 110, allowing effective immobilization of analytes from an aqueous solution at the detection elements 120.

The material of at least one, and preferably of both of the detection elements 120 further comprises a molecule composition for immobilizing analytes at the hydrophilic surface of the respective detection element 120.

More specifically, said molecule compositions are configured such that, when the microparticle 100 is placed in a fluid, such as an aqueous fluid, analytes comprised in the fluid are immobilized at the molecule compositions at the hydrophilic surfaces.

For this purpose, the molecule composition of at least one, and preferably of each of the detection elements comprises immobilization molecules of at least one type, for example proteins, antibiotics, antigens, lectins, biotin, streptavidin, aptamers, or enzymes.

In the depicted embodiment, the detection elements 120 are each formed from a mixture of the hydrophilic polymer and said molecule composition. Consequently, the detection elements 120 comprise the molecule compositions not only at the hydrophilic surfaces, but also in their bulk materials.

The molecule compositions of the detection elements 120 differ.

According to an embodiment, the detection elements 120 comprise the same type of immobilization molecules, but at different concentrations.

A respective embodiment allows to realize an assay with a wide dynamic range. After having introduced the microparticle 100 into the fluid with the analytes, the detection element 120 with the most suitable amount of analyte's binding sites can be used for the subsequent measurements and analysis, improving the accuracy of the assay.

In a similar embodiment, one of the detection elements 120 comprises the immobilization molecule at a significant concentration, whereas the immobilization molecule is essentially absent in another one of the detection elements 120.

In a respective embodiment, the other detection element 120 without any significant concentration of the immobilization molecules serves as a reference, for example when an assay is performed using the microparticle 100.

According to another, preferred embodiment, one of the detection elements 120 (or the molecule composition thereof, respectively) comprises immobilization molecules of a first type for immobilizing analytes of a first type, and another one of the detection elements 120 comprises immobilization molecules of a second type different from the first type for immobilizing analytes of a second type different from the first type.

Respective embodiments enable providing a multiplexed immunoassay platform, i.e., when an assay is performed using a respective microparticle 100, several analytes are selectively localized at the different detection elements 120 and thus become separately available at significant concentration for further analysis and measurements.

The microparticle 100 has a flattened shape, i.e., the thickness of the microparticle 100 along the z-axis is smaller than the width of the microparticle 100 along any direction in a plane x-y perpendicular to the z-axis along which the thickness of the microparticle 100 is defined.

Since the thickness t of the microparticle 100 is defined along the z-axis (i.e., along the direction along which the spatial extension of the microparticle 100 is the smallest), this direction is referred to as the thickness direction of the microparticle 100.

In the context of this disclosure, the wider sides of the microparticle 100 are also referred to as the top surface and the bottom surface of the microparticle 100. Notably, the terms bottom surface and top surface are used interchangeably throughout this disclosure. In other words, the terms "top" and "bottom" in the words top surface and bottom surface refer to the depiction of the microparticle 100 in the figures, and can be interchanged, e.g., by flipping the orientation of the figure, or by turning the depicted x, y, z coordinate system. In yet other words, the terms top surface and bottom surface do not imply an orientation of the respective surfaces with respect to a real-world reference frame, except for where the orientation of the particle with respect to the real-world reference frame is made explicit in the claims or in the following description.

When the microparticle 100 is used for analysis, for example in an assay, it is desirable to know the orientation of the microparticle 100 and thus the positions of the detection elements 120 relative to the position of the microparticle 100, for example relative to its center position or center-of-mass position.

Due to its flattened shape, when the microparticle 100 is used in the fluid, for example for an assay, the microparticle 100 will align with its wider sides facing up and down, and its thickness direction facing up, i.e., being orientated along the direction of the gravitational field. This orientation brings the center of mass of the microparticle 100 to the lowest possible position in the gravitational field of the earth, thus posing an energetically favorable arrangement. The remaining degrees of freedom of the microparticle 100 are the rotation around the direction of the gravitational field of the earth, and the binary degree of freedom as to whether the top surface of the bottom surface of the microparticle 100 faces up with respect to the gravitational field of the earth.

Conventional microparticles are formed with a highly symmetric shape, since a microparticle with a highly symmetric shape is technically easier to produce than a microparticle having a lower symmetry. For example, when the conventional microparticle is produced using stop-flow lithography, its highly symmetric shape allows for the usage of a fluid channel or several interconnected fluid channels having a highly symmetric cross-section, such as a circular cross-section, a rectangular cross-section, or a square shaped cross-section.

As a consequence of the highly symmetric shape of a conventional microparticle, when the conventional microparticle aligns with its wider sides facing up and down, and its thickness direction facing up as described above for the microparticle 100, the two remaining degrees of freedom (rotation around the direction of the gravitational field of the earth, binary degree of freedom) give rise to an uncertainty about the orientation of the conventional microparticle. For example, if a conventional microparticle had a flattened cylindrical shape, observation of this conventional microparticle aligned as explained above under a microscope, which typically views along (or opposite to) the earth's gravitational field cannot determine the azimuthal angle related to the rotation of the conventional microparticle around the direction of the earth's gravitational field. A respective observation cannot determine either, whether the top surface or the bottom surface of the conventional microparticle faces upwards in the earth's gravitational field.

In contrast, the microparticle 100, and more specifically, the body 110 of the microparticle 100 according to the embodiment of Fig. 1a, Fig. 1b has a low-symmetry shape. In other words, the microparticle 100, or its body 110, respectively, does not have any mirror symmetry about any mirror plane other than the x-y plane intersecting all the detection elements 120 at a position along the z axis corresponding to half the thickness t of the microparticle 100.

Hence, when the microparticle 100 according to the embodiment of Fig. 1a, Fig. 1b is allowed to align in the earth's gravitational field and is then observed along the direction of the earth's gravitational field, for example using a microscope, this observation immediately and fully determines the orientation of the microparticle 100. A respective observation thus determines uniquely and unambiguously the positions of the detection elements 120 with respect to the microparticle 100, which may, for example be expressed in terms of relative positions with respect to the circumference of the microparticle 100 or its body 110, or in terms of relative positions with respect to the center of mass of the microparticle 100 or of its body 110, respectively.

In particular, since the microparticle 100, or its body 110, respectively, does not have any mirror symmetry about any mirror plane other than the x-y plane intersecting all the detection elements 120, the shape of the microparticle 100 is chiral, and it is thus immediately obvious from the observation explained above whether the top surface or the bottom surface of the microparticle 100 faces upwards with respect to the earth's gravitational field.

In the example of Fig. 1a, Fig. 1b this becomes obvious when following the circumference of the microparticle 100, or its body 110, respectively, in a clockwise direction. Starting from the topmost position along the circumference, this topmost position is part of the longest edge of the circumference. Along the clockwise direction, an acute angle and a shorter edge follow, followed by an optuse angle and an even shorter edge. If the microparticle 100 was flipped upside down, this order of elements would be reversed, or would be observed along the counterclockwise direction, respectively.

In other words, a user performing an assay or another optical measurement on the microparticle 100 immediately knows that the detection element 120 closer to the aforementioned acute angle is the first detection element, and the detection element 120 closer to the aforementioned obtuse angle is the second detection element, independent of the orientation (i.e., rotation about the z axis and up/down-flipping) of the microparticle 100.

This is particularly beneficial as the detection elements 120 comprise different molecule compositions for the immobilization of the analytes. Knowing which molecule composition is associated with which of the detection elements 120, the user can immediately identify which of the detection elements 120 carries which molecule composition.

Fig. 2a, Fig. 2b show an embodiment of a microparticle similar to the microparticle 100 of Fig. 1a, Fig. 1b. Similar elements are described with identical reference numerals. To avoid repetition, they will not be described again. Instead, the following description focuses on the modifications over the foregoing embodiment.

In Fig. 2a, Fig. 2b, the thickness t and the width w of the microparticle 100 are indicated.

Preferably, the width w is at least 1.5 times as large as the thickness t, and more preferably at least twice as large as the thickness t, and even more preferably at least 2.5 or at least three times as large as the thickness t.

The inventors have realized that a respective aspect ratio between width w and thickness t beneficially ensures a reliable orientation of the microparticle 100 with its top surface or bottom surface pointing up when the microparticle 100 is allowed to align in the earth's gravitational field.

Each of the detection elements 120 of the microparticle 100 of Fig. 2a, Fig. 2b comprises a cavity 122 in the form of a through hole extending from the top surface of the microparticle 100 to its bottom surface.

On the one hand, the cavity 122 ensures that an analyte which enters therein is surrounded from several sides by the hydrophilic surface of the detection element 120, improving the probability of successful immobilization of the analyte.

In addition, the through hole in the detection element 120 beneficially allows for measurements and analysis of analytes immobilized at the detection element 120 in the cavity 122 in transmittance geometry.

The cavity 122 is particularly beneficial for cell encapsulation in the microparticle 100 to perform a cell-section assay. Similar to analyzing biofluid from patients, proteins are also secreted by cells. By adding cell-attachment motifs to the detection element 120, cells can entrap in the cavity 122 of the detection element 120, which will then release proteins, that are detected by similar same ELISA steps.

According to some embodiments, the first/second molecule composition is adapted to provide analyte binding sites and are mixed in the fluid streams that the detection elements 120 are formed from before the fabrication of the microparticle 100 as described below. In respective embodiments, the first/second molecule composition is cross-linked in the fabrication process all over the detection element 120 at some specific concentration. Therefore, a microparticle 100 without any cavity 122 can immobilize analyte on a top and bottom surface of the respective detection element 120, where the detection element 120 is in direct contact with the fluid.

Provision of the cavity 122 enlarges the contact area between the microparticle 100 and the fluid, so that more potential binding sites are provided.

For example, for a round detection element 120 without a cavity 122, the provided contact area is pi*r² at both the top surface and the bottom surface of the detection element 120 embedded in the body, where r is the radius of the round detection element 120. Providing the detection element 120 with the cavity 122 in the form of a through hole enlarges the contact area by the area of the side wall of the through hole, which is 2*pi*r*t. In an exemplary embodiment, the thickness t is 150 µm, and the radius r = 60 µm.

Fig. 3a, Fig. 3b show an embodiment of a microparticle similar to the microparticle 100 according to the foregoing embodiments. Similar elements are indicated with identical reference numerals. To avoid repetition, they will not be described again. Instead, the following description focuses on the modifications over the foregoing embodiments.

In contrast to the microparticles 100 according to the foregoing embodiments, the microparticle 100 according to the embodiment of Fig. 3a, Fig. 3b has a highly symmetric outer shape, or circumferential shape, respectively.

To break the mirror symmetry of the microparticle 100, the detection elements 120 comprise different marker materials having different optical properties. For this purpose, in the depicted embodiment, the detection elements 120 each comprise a different fluorescent dye.

Thus, when the microparticle 100 is allowed to align in the earth's gravitational field and is then observed with a microscope along the direction of the earth's gravitational field, the orientation of the microparticle 100 can beneficially be determined by performing a fluorescence microscopy measurement with the microscope.

The microparticle 100 according to the embodiment of Fig. 3a, Fig. 3b has further been modified over the microparticles 100 according to the foregoing embodiments to comprise three detection elements 120. This improves the multiplexing capabilities of the microparticle 100, for example when the microparticle 100 is used in an assay as described above.

Fig. 4a, Fig. 4b show an embodiment of a microparticle similar to the microparticle 100 according to the foregoing embodiments. Similar elements are indicated with identical reference numerals. To avoid repetition, they will not be described again. Instead, the following description focuses on the modifications over the foregoing embodiments.

The microparticle 100 according to the depicted embodiment comprises a marker structure 112 associated with the outer surface of the body 110, breaking the mirror symmetry of the microparticle 100. More specifically, the marker structure 112 breaks the mirror symmetry of the outer shape, or of the circumferential shape, respectively, of the microparticle 100.

In the depicted embodiment, the marker structure 112 comprises a plurality of at least three recesses, and in the depicted embodiment exactly three recesses, arranged at unequal distances on the outer surface of the body 110, i.e., the distance between a first pair of recesses from the plurality of at least three recesses is different from the distance between a second pair of recesses from the plurality of at least three recesses.

When the microparticle 100 is allowed to align in the earth's gravitational field and is then observed with a microscope along the direction of the earth's gravitational field, the orientation of the microparticle 100 can beneficially be determined by performing a bright-light observation of the outer shape of the microparticle 100 with the microscope.

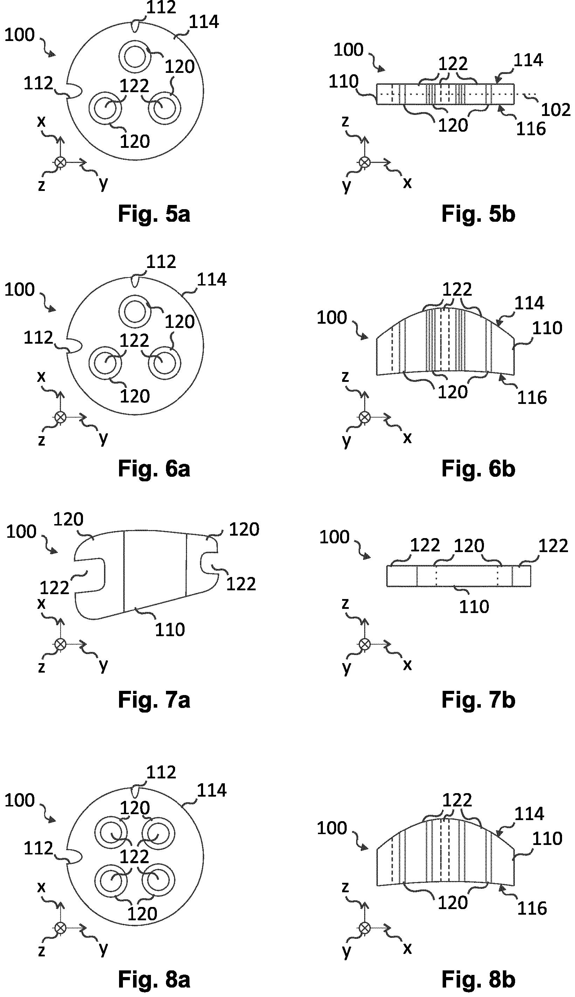

Fig. 5a, Fig. 5b show an embodiment of a microparticle similar to the microparticle 100 according to the foregoing embodiments. Similar elements are indicated with identical reference numerals. To avoid repetition, they will not be described again. Instead, the following description focuses on the modifications over the foregoing embodiments.

The microparticle 100 according to the depicted embodiment comprises the marker structure 112 associated with the outer surface of the body 110, breaking the mirror symmetry of the microparticle 100. More specifically, the marker structure 112 breaks the mirror symmetry of the outer shape, or of the circumferential shape, respectively, of the microparticle 100.

In the depicted embodiment, the marker structure 112 comprises two recesses with different sizes on the outer surface of the body 110.

In the depicted embodiment, the marker structure 112 comprises two recesses with different shapes on the outer surface of the body 110.

When the microparticle 100 is allowed to align in the earth's gravitational field and is then observed with a microscope along the direction of the earth's gravitational field, the orientation of the microparticle 100 can beneficially be determined by performing a bright-light observation of the outer shape of the microparticle 100 with the microscope.

Fig. 6a, Fig. 6b show an embodiment of a microparticle similar to the microparticle 100 according to the foregoing embodiments. Similar elements are indicated with identical reference numerals. To avoid repetition, they will not be described again. Instead, the following description focuses on the modifications over the foregoing embodiments.

While the microparticles 100 according to the foregoing embodiments have parallel top and bottom surfaces, the top surface and the bottom surface of the microparticle 100 according to the embodiment of Fig. 6a, Fig. 6b are not parallel. Both configurations are possible, although the parallel top and bottom surfaces are preferred, as they provide an improved reliability of the alignment of the microparticle 100 in the earth's gravitational field (i.e., with the top surface or the bottom surface pointing up).

While the microparticles 100 according to the foregoing embodiments have flat top and bottom surfaces, the top surface and the bottom surface of the microparticle 100 according to the embodiment of Fig. 6a, Fig. 6b are not flat. Both configurations are possible, although the flat top and bottom surfaces are preferred, as they provide an improved reliability of the alignment of the microparticle 100 in the earth's gravitational field (i.e., with the top surface or the bottom surface pointing up).

Fig. 7a, Fig. 7b show an embodiment of a microparticle 100 similar to the microparticle 100 according to the foregoing embodiments. Similar elements are described with identical reference numerals. To avoid repetition, they will not be described again. Instead, the following description focuses on the modifications over the foregoing embodiments.

While in the microparticles 100 according to the foregoing embodiments, the detection elements 120 are embedded in the body 110, this is not the case for the microparticle 100 according to the embodiment of Fig. 7a, Fig. 7b.

Both configurations are possible, although the detection elements 120 embedded in the body 110 are preferred, as they provide an improved structural integrity of the microparticle 100.

Fig. 8a, Fig. 8b show an embodiment of a microparticle 100 similar to the microparticle 100 according to the foregoing embodiments. Similar elements are described with identical reference numerals.

The microparticle 100 according to the embodiment of Fig. 8a, Fig. 8b comprises four detection elements. This improves the multiplexing capabilities of the microparticle 100, for example when the microparticle 100 is used in an assay as described above.

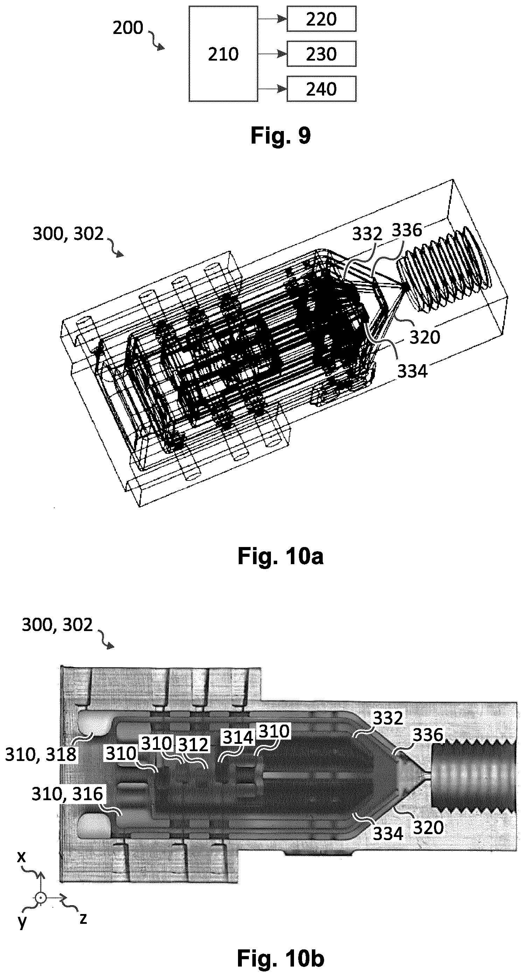

Fig. 9 illustrates a method 200 for fabricating the microparticle 100 described above.

The method 200 comprises providing 210 parallel flows of fluids in a flow channel, said parallel flows of fluids comprising a flow of a fluid comprising a precursor for a hydrophobic material, a flow of a first additional fluid, the first additional fluid comprising a precursor for a hydrophilic material, and a flow of a second additional fluid, the second additional fluid comprising a precursor for a hydrophilic material.

The method 200 comprises curing 220 the precursor for the hydrophobic material into a hydrophobic material forming at least part of the body 110.

The method 200 comprises curing 230 the precursor for the hydrophilic material comprised in the first additional fluid into a hydrophilic material forming at least part of the first detection element.

The method 200 comprises curing 240 the precursor for the hydrophilic material comprised in the second additional fluid into a hydrophilic material forming at least part of the second detection element.

Fig. 10a to Fig. 10n illustrate an apparatus 300 for fabricating the microparticle 100 described above, or for carrying out the method 200 described above, respectively.

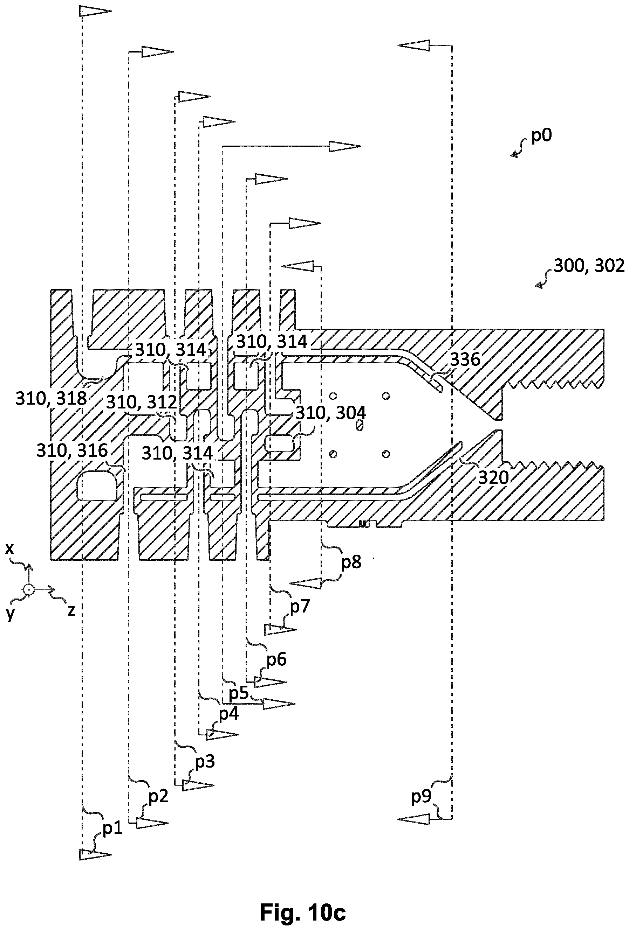

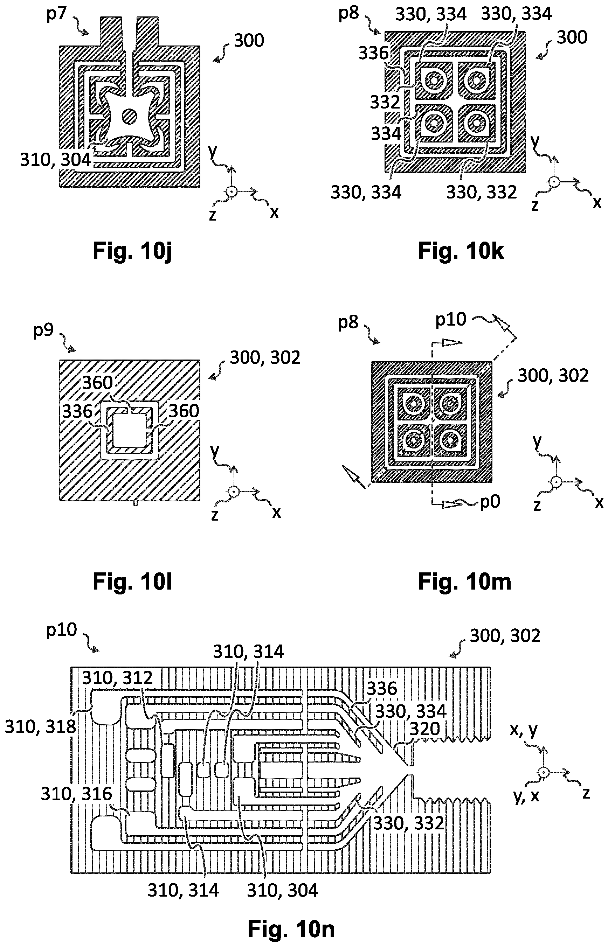

Therein, Fig. 10a gives a perspective view of a three-dimensional model of the apparatus 300. Fig. 10b gives a photograph of a prototype corresponding to the three-dimensional model, wherein the photograph has been recorded in bright-field microscopy. Fig. 10c to Fig. 10n give cross-sectional views through the apparatus 300, as indicated by planes p0 to p10 indicated in Fig. 10c and in Fig. 10m.

The apparatus 300 comprises a flow channel 302. The flow channel 302 defines a flow direction for the parallel flows of the fluids therein. In the depicted embodiment, the coordinate system is orientated such that the z-axis coincides with the flow direction. The flow channel 302 comprises walls enclosing the parallel flows of the fluids in the plane perpendicular to the flow direction, which is the x-y plane in the depicted embodiment.

In the following, the apparatus 300, or the flow channel 302, respectively is described following along the z direction, or along the downstream direction of the flow channel 302, respectively.

In the upstream part of the flow channel 302, several inlet openings 310 are arranged inside of the flow channel 302, as is best visible in Fig. 10a to Fig. 10c and in Fig. 10m.

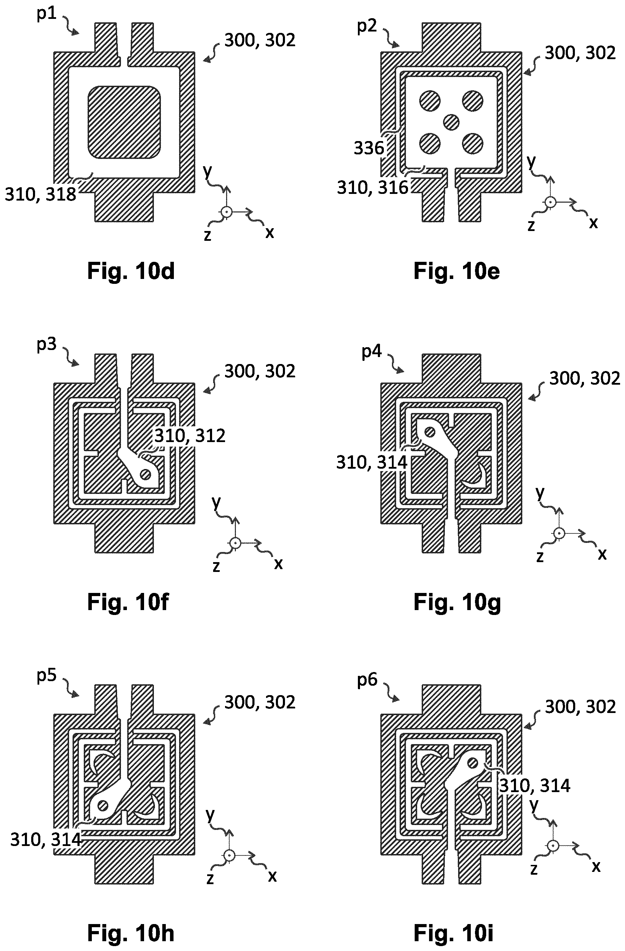

Amongst the inlet openings 310, most upstream, a second outer inlet opening 318 with a large width is provided, as is best visible in Fig. 10d showing a cross section in plane p1.

The second outer inlet opening 318 serves to provide a carrier flow, or a flow of a carrier fluid, respectively, into the flow channel 302. This carrier flow is essentially free of precursors, i.e., it comprises significantly less (i.e., in terms of a molar concentration) of the hydrophobic precursor than the flow of the fluid comprising the precursor for a hydrophobic material described above in the context of the method 200, and it comprises significantly less (i.e., in terms of a molar concentration) of the precursor for the hydrophilic material than the flow of the first additional fluid and than the flow of the second additional fluid. Thus, when the aforementioned precursors are cured to form solid materials, essentially no solid material is formed in the carrier flow. Due to its large width, the carrier flow thus forms a liquid carrier flow around the formed solids, even if the carrier flow is or was exposed to UV illumination. The carrier flow thus serves to transport the formed solids and to prevent clogging of the flow channel 302.

Amongst the inlet openings 310, next (i.e., along the downstream direction, or along the positive z direction, respectively), a first outer inlet opening 316 with a width smaller than the one of the second outer inlet opening 318 is provided in the flow channel 302, as is best visible in Fig. 10e showing a cross section in plane p2.

This first outer inlet opening 316 serves to provide the flow of the fluid comprising the precursor for the hydrophobic material described above in the context of the method 200.

In other words, the first outer inlet opening 316 serves to provide the precursor that the material of the body 110 of the microparticle 100 is to be formed from.

In the upstream portion of the flow channel 302, the first outer inlet opening 316 is separated from the flow of the fluid from the first outer inlet opening 316, or from the carrier flow, respectively, by an outer tapered element 336.

In the upstream portion of the flow channel 302, the outer tapered element 336 surrounds the first outer inlet opening 316 in a plane (x-y plane) perpendicular to the flow direction (z direction) defined by the flow channel 302, and the flow of the fluid from the second outer inlet opening 318 (carrier flow) surrounds the outer tapered element 336 in said plane.

Despite being referred to as the outer "tapered" element 336, the outer tapered element 336 is not necessarily tapered in this upper section of the flow channel 302. In the depicted embodiment, the outer tapered element 336 instead maintains its width, or diameter, respectively, in the section of the flow channel 302 with the inlet openings 310. In the depicted embodiment, the outer tapered element 336 only tapers in a section of the flow channel 302 downstream from the inlet openings 310.

Amongst the inlet openings 310, next (i.e., along the downstream direction), a first inlet opening 312 is provided in the flow channel 302, as is best visible in Fig. 10f showing a cross section in plane p3.

This first inlet opening 312 serves to provide the flow of the first additional fluid described above in the context of the method 200.

In other words, this first inlet opening 312 serves to provide the material that a first one of the detection elements 120 of the microparticle 100 is to be formed from.

The first inlet opening 312 is offset from the center of the flow channel 302 in the plane (x-y plane) perpendicular to the flow direction (z direction).

The first inlet opening 312 is surrounded by the outer tapered element 336, by the flow of the fluid from the second outer inlet opening 318 (carrier flow), and by the flow of the fluid from the first outer inlet opening 316, namely in the plane (x-y plane) perpendicular to the flow direction (z direction).

Amongst the inlet openings 310, next (i.e., along the downstream direction), at least one second inlet opening 314 is provided in the flow channel 302.

In the depicted embodiment, the at least one second inlet opening 314 comprises a second inlet opening 314 (shown, e.g., in Fig. 10g showing a cross section in plane p4), a third inlet opening 314 (shown, e.g., in Fig. 10h showing a cross section in plane p5), and a fourth inlet opening 314 (shown, e.g., in Fig. 10i showing a cross section in plane p6). Thus, three respective inlet openings 314 are provided in addition to the first inlet opening 312.

In alternative embodiments (not shown), the at least one second inlet opening 314 comprises only the second inlet opening 314 (shown, e.g., in Fig. 10g) and the third inlet opening 314.

In alternative embodiments (not shown), the at least one second inlet opening 314 comprises only the second inlet opening 314 (shown, e.g., in Fig. 10g).

The second inlet opening 314 serves to provide the flow of the second additional fluid described above in the context of the method 200. In other words, the second inlet opening 314 serves to provide the material that a second one of the detection elements 120 of the microparticle 100 is to be formed from.

In embodiments with a third inlet opening 314, the third inlet opening 314 serves to provide a flow of a third additional fluid (similar to the flow of the first additional fluid and/or to the flow of the second additional fluid described above in the context of the method 200). In other words, the third inlet opening 314 serves to provide the material that a third one of the detection elements 120 of the microparticle 100 is to be formed from.

In embodiments with a fourth inlet opening 314, the fourth inlet opening 314 serves to provide a flow of a fourth additional fluid (similar to the flow of the first additional fluid and/or to the flow of the second additional fluid described above in the context of the method 200). In other words, the fourth inlet opening 314 serves to provide the material that a fourth one of the detection elements 120 of the microparticle 100 is to be formed from.

In embodiments, wherein the at least one second inlet opening 314 are actually several second inlet openings 314, such as in the depicted embodiment having three second inlet openings 314, the respective second inlet openings 314 are each laterally offset from each other and from the first inlet opening 312 in the plane (x-y plane) perpendicular to the flow direction (z direction).

Amongst the inlet openings 310, next (i.e., along the downstream direction), an inner inlet opening 304 is provided in the flow channel 302, as is best visible in Fig. 10j showing a cross section in plane p7.

The inner inlet opening 304 serves to provide an inner flow of a fluid essentially free of precursors, similar to the carrier fluid (or to the carrier flow, respectively) described above. Thus, when the precursors are cured to form solid materials, essentially no solid material is formed in the inner flow.

The inner flow thus serves to provide the cavities 122 of the microparticle 100.

Fig. 10k shows a cross section through the flow channel 302 in the plane p8 after the last one of the at least three inlet openings 310, or, more specifically, just after the inner inlet opening 304.

Up to this plane p8 (i.e., along the downstream direction), both the flow channel 302 and the outer tapered element 336 have a constant width, or a constant cross-sectional area, respectively.

Fig. 10l shows a cross section through the flow channel 302 in the plane p9 in a reducing section 320 of the flow channel 302 downstream from the inlet openings 310.

As is visible from Fig. 10l, and also in Fig. 10a - Fig. 10c and Fig. 10n, in the reducing section 320, the cross-sectional area (or the width, respectively) of both the flow channel 302 and of the outer tapered element 336 decreases in the downstream direction.

Thus, the pattern formed by the parallel flows of the fluids in the flow channel 302 is "compressed" into a pattern with a smaller cross-section area (or the width, respectively).

The flow channel 302 according to the depicted embodiment thus allows for sculpting the pattern formed by the parallel flows of the fluids in the flow channel 302 upstream of the reducing section 320 using inlet openings 310 with sizes on a scale of several millimeters, and only thereafter compressing the pattern into a much smaller structure for the microparticle 100.

This approach permits to shape the microparticle 100 with a high (submillimeter) accuracy, without a need to manufacture the inlet openings 310 with the same level of accuracy.

Also shown in Fig. 10l are notches 360 formed in the outer tapered element 336. These notches 360 shape the flow of the fluid from the first outer inlet opening 316 (i.e., the fluid comprising the precursor for the hydrophobic material for forming the body 110 of the microparticle 100) to provide the marker structures 112 in the form of recesses 112, as described above in the context of the microparticle 100 according to Fig. 4a -Fig. 6b, Fig. 8a, and Fig. 8b .

Fig. 10m repeats the cross-sectional view already depicted in Fig. 10k, with an indication of the plane p0 (shown in Fig. 10c) and of the plane p10 (shown in Fig. 10n).

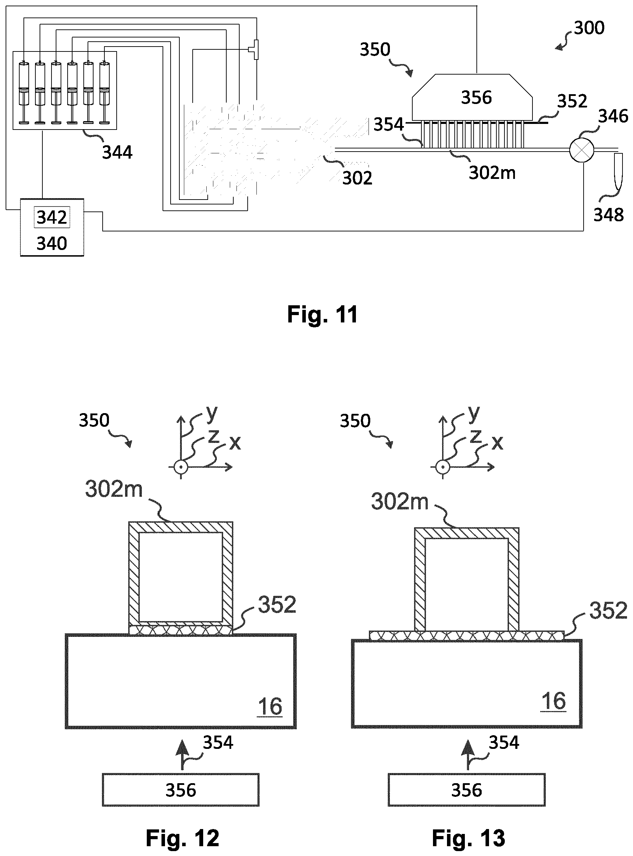

Fig. 11 illustrates an embodiment of an apparatus 300 similar to the apparatus 300 according to the foregoing embodiment. Similar elements are indicated with identical reference numerals. To avoid repetition, they will not be described again. Instead, the following description focuses on the modifications over the foregoing embodiment.

In addition to the flow channel 302 described in detail above, the apparatus 300 of Fig. 11 comprises a pump and reservoir system 344 for providing the aforementioned fluids to the inlet openings 310.

In the depicted embodiment, the pump and reservoir system 344 comprises a plurality of syringe pumps, including at least one syringe pump for providing each of the aforementioned fluids.

The apparatus 300 according to the embodiment of Fig. 11 further comprises a lithographic masking section 350.

A section 302m of the flow channel 302 passes through the lithographic masking section 350.

A lithographic mask 352 is arranged in the lithographic masking section 350 above said section 302m of the flow channel 302.

A UV source 356 is arranged in the lithographic masking section 350 above the lithographic mask 352.

The apparatus 300 of Fig. 11 further comprises a valve 346 for selectively shutting off the flow channel 302, or for selectively shutting off the parallel flows of the fluids in the flow channel 302, respectively. In other words, the valve 346 is adapted to selectively stop the parallel flows of the fluids in the flow channel 302.

The apparatus 300 of Fig. 11 further comprises a particle collection system 348. In the depicted embodiment, the particle collection system 348 comprises a filter for filtering microparticles 100 out of a stream of fluids through the flow channel 302.

In the depicted example of the particle collection system 348, the microparticles 100 are collected in a commercially available 15 ml falcon tube. Then a washing buffer (ethanol/water/PBS) is added, which results in diluting the uncured material and making it less dense compared to the cured microparticles 100 which settle down. The uncured material is removed and the process is repeated with fresh wash solution 3 times.

The apparatus 300 of Fig. 11 further comprises a computer system 340 comprising controllers 342 for controlling the pump and reservoir system 344, the UV source 356, and the valve 346.

To fabricate particles, the computer system 340 controls the other components of the apparatus 300 in the following manner:

In a first step, the computer system 340 controls the valve 346 to be in an open state, and the computer system 340 controls the pump and reservoir system 344 to provide the aforementioned fluids (i.e., the fluid comprising the precursor for the hydrophobic material, the first additional fluid, and the second additional fluid as explained above in the context of the method 200; and in embodiments with the carrier flow also the carrier fluid; and in embodiments with the inner flow, also the fluid of the inner flow) to the at least three inlet openings 310 of the flow channel 302.

In response to this first step, the parallel flows of the fluids (as described above in the context of the method 200) are provided in the flow channel 302 via the at least three inlet openings 310.

In a second step after the first step, the computer system 340 controls the valve 346 to be in a closed state. Optionally, in the second step, the computer system 340 controls the pump and reservoir system 344 to stop providing the aforementioned fluids. In an alternative embodiment, the pump and reservoir system 344 remains switched on, and only the valve 346 is controlled to be in a closed state.

In response to this second step, the parallel flows of the fluids (as described above in the context of the method 200) are stopped in the flow channel 302, 302m.

In a third step after the second step, the computer system 340 controls the UV source 356 to produce UV radiation 354.

In response to this third step, the UV source 356 shines the UV radiation 354 through the lithographic mask 352 and into the section 302m of the flow channel 302.

Under the resulting irradiation with UV radiation 354, the precursor for the hydrophobic material, the precursor for the hydrophilic material comprised in the first additional fluid, and the precursor for the hydrophilic material comprised in the second additional fluid (and in embodiments with a third and/or a fourth additional fluid, also the precursors for the hydrophilic material comprised in the third and/or fourth additional fluid) get cured, i.e., photochemically cured. In other words, those precursors solidify under the exposure to the UV radiation 354, such that the precursor for the hydrophobic material solidifies into material of the body 110, the precursor for the hydrophilic material comprised in the first additional fluid solidifies into material of a first detection element 120, and the precursor for the hydrophilic material comprised in the second additional fluid solidifies into material of a second detection element 120 (and, in embodiments with a third and/or a fourth additional fluid, the precursor for the hydrophilic material comprised in the third and/or the fourth additional fluid solidifies into material of a third and/or a fourth detection element 120).

Notably, the first additional fluid is provided with the molecule composition of the first detection element 120 as described above in the context of the microparticle 100 according to the embodiments of Fig. 1a to Fig. 8b. Thus, the molecule composition comprised in the first additional fluid is comprised in the material of a first detection element 120 formed therefrom.

Correspondingly, the second additional fluid is provided with the second molecule composition of the second detection element 120 as described above. Thus, the molecule composition prized in the second additional fluid is comprised in the material of a second detection element 120 formed therefrom.

In embodiments with a third and/or a fourth additional fluid, the third/fourth additional fluid is provided with the third/fourth molecule composition of the third/fourth detection element 120 as described above. Thus, the molecule composition comprised in the third/fourth additional fluid is comprised in the material of a third/fourth detection element 120 formed therefrom.

In a fourth step after the third step, the computer system 340 controls the UV source 356 to stop producing the UV radiation 354.

The computer system 340 then controls the apparatus 300 to iterate the process, starting again from the first step.

Fig. 12 and Fig. 13 illustrate lithographic masking sections 350 of the apparatus 300 according to two different embodiments.

In the embodiment of Fig. 12, the lithographic mask 352 is arranged on a substrate 16, and the section 302m of the flow channel 302 is arranged over/above the lithographic mask 352.

The substrate 16 is at least partially transparent to the UV radiation 354 from the UV source 356. Thus, the UV radiation 354 passes through the substrate 16, through slits (not shown) in the mask 352, and into the section 302m of the flow channel 302.

During operation, the aforementioned fluids with the precursors flow are located in the section 302m of the flow channel 302. Upon irradiation with the UV radiation 354, the precursors are cured/solidify to form solid material of the body 110 and of the detection elements 120.

The embodiment of Fig. 13 is similar to the embodiment of Fig. 12. However, in the embodiment of Fig. 13, the wall of the flow channel 302 is thinner or even absent in a region adjacent to the lithographic mask 352. Hence, the fluid is constrained by the lithographic mask 352 and/or by the substrate 16 in the respective region.

In the embodiments depicted in Fig. 12, Fig. 13, the section 302m of the flow channel 302 in the lithographic masking section 350 has a rectangular or quadratic cross-section. In alternative embodiments, the section 302m of the flow channel 302 in the lithographic masking section 350 has an ellipsoidal or, preferably, round cross-section (not shown).

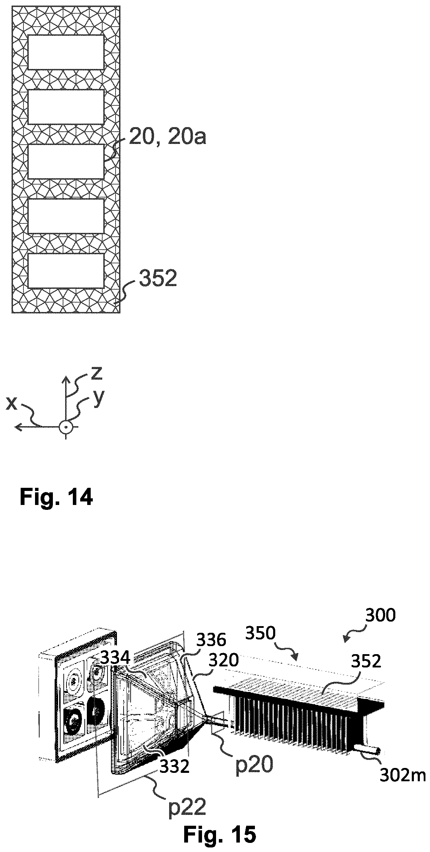

Fig. 14 illustrates a lithographic mask 352 of the apparatus 300 according to an embodiment.

The lithographic mask 352 according to the embodiment of Fig. 14 comprises slits 20 having segments 20a with an extension (along the x-direction) perpendicular to a flow direction (corresponding to the z direction) defined by the flow channel 302 in the lithographic masking section 350.

Fig. 15 schematically illustrates a lithographic masking section 350 and a reducing section 320 of a flow channel 302, of an apparatus 300 according to an example of the present disclosure.

In Fig. 15, elements similar to elements described above are indicated with the same reference numerals. For a detailed description of the respective elements, reference is made to their detailed description above.

Further, planes p20 (between the reducing section 320 and the lithographic masking section 350, or at the transition between the reducing section 320 and the lithographic masking section 350, respectively) and p22 (at the upstream end of the reducing section 320) are indicated in Fig. 15.

According to the embodiment of Fig. 15, the section 302m of the flow channel 302 in the lithographic masking section 350 has a circular cross-section.

Notably, the square- and ring-shaped elements to the left of the reducing section 320 in Fig. 15 depict the parallel flows of the fluids in the flow channel 302 upstream of the reducing section 320 (rather than physical structures of the flow channel 302 itself), similar to the parallel flows of the fluids shown schematically in Fig. 10k.

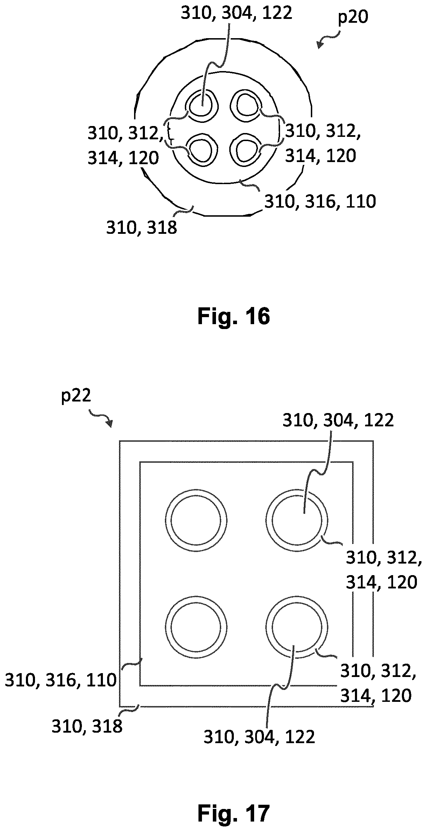

Fig. 16 shows a cross section through the parallel flows of the fluids in the flow channel 302 in a plane p20 between the reducing section 320 and the lithographic masking section 350, or at the transition between the reducing section 320 and the lithographic masking section 350, respectively. The cross section through the parallel flows of the fluids in the flow channel 302 is similar in the section 302m of the flow channel 302 in the lithographic masking section 350.

Reference numerals in Fig. 16 indicate the inlet that the respective parallel flow of the fluid in the flow channel 302 is provided from. For a detailed description of the indicated inlets and the provided parallel flows of the fluids, reference is made to the respective detailed description in the context of Fig. 10a to Fig. 10n.

Fig. 17 shows a cross section through the parallel flows of the fluids in the flow channel 302 in a plane p22 at the upstream end of the reducing section 320.

Reference numerals in Fig. 17 indicate the inlet that the respective parallel flow of the fluid in the flow channel 302 is provided from. For a detailed description of the indicated inlets and the provided parallel flows of the fluids, reference is made to the respective detailed description in the context of Fig. 10a to Fig. 10n.

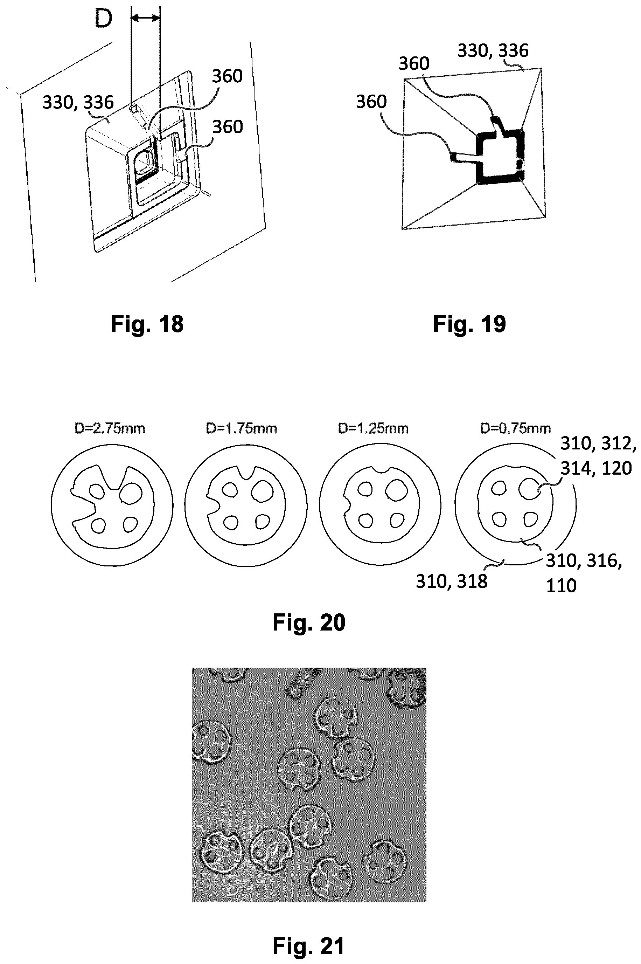

Fig. 18 and Fig. 19 depict two different examples of outer tapered elements 336.

The detailed description of the outer tapered elements 336 given above applies also to the tapered elements 336 of Fig. 18 and Fig. 19. The following description focuses on modifications implemented in the tapered elements 336 of Fig. 18 and Fig. 19.

The tapered elements 336 of Fig. 18 and Fig. 19 are provided with notches 360 each having a length D, referring to the length D of the notches along the flow direction defined by the flow channel 302. In the depicted embodiments, the length D refers to the length as measured from the downstream end of the tapered element 336.

In the depicted embodiments, the tapered elements 336 is provided with two notches 360 having different lengths D, resulting in the fabrication of a microparticle 100 similar to the one of Fig. 5a, Fig. 5b. In the specific example, one of the notches 360 has a length D of 1.5 mm, and the other notch has a length D of 0.5 mm.

In alternative embodiments (not shown), the tapered element 336 is provided with at least three notches 360. In respective embodiments, distances between neighboring ones of the at least three notches 360 differ, resulting in the fabrication of a microparticle 100 similar to the one of Fig. 4a, Fig. 4b. In respective embodiments, the lengths of the at least three notches 360 may be the same or they may be different.

Fig. 20 shows cross sections through the parallel flows of the fluids in the flow channel 302 in a plane p20 between the reducing section 320 and the lithographic masking section 350, or at the transition between the reducing section 320 and the lithographic masking section 350, respectively; similar to the presentation in Fig. 16. As in Fig. 16, reference numerals in Fig. 20 indicate the inlet that the respective parallel flow of the fluid in the flow channel 302 is provided from. For a detailed description of the indicated inlets and the provided parallel flows of the fluids, reference is made to the respective detailed description in the context of Fig. 10a to Fig. 10n.

In Fig. 20, the cross sections through the parallel flows of the fluids are given for different apparatus, each having a tapered element 336 each with two notches 360 (as depicted in Fig. 18, Fig. 19) of equal, lengths D. Those lengths D are different for the different apparatus 300. The lengths D are indicated for the different apparatus 300, with the lengths being 2.75 mm, 1.75 mm, 1.25 mm, and 0.75 mm.

Fig. 21 gives a microscopy image of microparticles 100.

The microparticles 100 are each formed with a first detection element 120 comprising a first fluorescent dye, with a second detection element 120 comprising a second fluorescent dye, with a third detection element 120 comprising a third fluorescent dye, and, with a fourth detection element 120 comprising a fourth fluorescent dye. Each of the first, second, third and fourth fluorescent dye is a different fluorescent dye, or has a different photoluminescence (or fluorescence, respectively) emission wavelength, respectively.

As can be seen from the different apparent brightnesses of the detection elements 120 within each of the microparticles 100, the individual detection elements 120 within each of the microparticles 100 can be distinguished and identified in the microscopy image, with the advantages described above in the context of the microparticles according to the embodiments of Fig. 1a to Fig. 8b.

Actually, the photoluminescence (or fluorescence, respectively) emission wavelengths from the first, second, third and fourth fluorescent dye differ, although this may not be apparent from the grayscale photograph, and hence the individual detection elements 120 within each of the microparticles 100 can be distinguished and identified based on their photoluminescence (or fluorescence, respectively) emission wavelengths, or colors, respectively.

Fig. 22 shows an apparatus 300 similar to the apparatus of Fig. 10a to Fig. 10n. Similar elements are indicated with identical reference numerals as in Fig. 10a to Fig. 10n. For a detailed description of those elements, reference is made to the corresponding description in the context of Fig. 10a to Fig. 10n.

In contrast to the apparatus 300 according to the embodiment of Fig. 10a to Fig. 10n, the flow channel 302 of the apparatus 300 according to the embodiment of Fig. 22 is formed with four inlet openings (rather than with seven inlet openings) 310, namely with the first outer inlet opening 316, with the second outer inlet opening 318, with the first inlet opening 312, and with the second inlet opening 314.

In other words, the apparatus 300 according to the embodiment of Fig. 22 does not comprise the third inlet opening, the fourth inlet opening, and the inner inlet opening.

Fig. 23 shows an apparatus 300 similar to the apparatus of Fig. 22. Similar elements are indicated with identical reference numerals. For a detailed description of those elements, reference is made to the corresponding description in the context of Fig. 10a to Fig. 10n.

The apparatus 300 of Fig. 23 is similar to the apparatus 300 of Fig. 22. As compared to the apparatus 300 of Fig. 22, the flow channel 302 of the apparatus 300 of Fig. 23 further comprises the inner inlet opening 304. For a detailed description of the inner inlet opening 304, reference is made to the corresponding description in the context of Fig. 10a to Fig. 10n.

Fig. 24 illustrates a process involving microparticle 100, detailing a sequence of steps for immobilizing analytes. The figure shows a series of stages, including biotinylation, streptavidin binding, and antibody immobilization, followed by biomarker detection and amplification. Each step is depicted with corresponding reagents and conditions, highlighting the complex interactions required to achieve successful analyte immobilization.

The microparticle 100 serves as the substrate for these interactions, providing hydrophilic surfaces for the binding and detection of analytes.

The detection elements 120 of the microparticle 100 of Fig. 24 are formed of hydrophilic material comprising biotin, i.e., from first and second additional fluids each comprising a precursor for a hydrophilic material and biotin (more specifically, acrylate-PEG-biotin) in addition. According to some embodiments, the concentration of biotin in the different detection elements 120 of the microparticle 100 differ. The inventors have successfully performed experiments to demonstrate that the measured signal intensity indeed increases with biotin concentration, in an approximately linear manner.

The steps shown in Fig. 24 follow a known protocol of sandwich ELISA, as previously reported in: Ghulam Destgeer, Mengxing Ouyang, Chueh-Yu Wu, and Dino Di Carlo: "Fabrication of 3D concentric amphiphilic microparticles to form uniform nanoliter reaction volumes for amplified affinity assays," Lab on a Chip, 2020, 20, 3503-3514. This document is incorporated herein in its entirety by reference.

The sequence of steps shown in Fig. 24 demonstrates the functionality of the microparticle 100 in a practical application. Each stage of the process builds upon the previous one, ultimately leading to the amplification with oil encapsulation and detection of the target analytes. This process highlights the importance of the microparticle's 100 design and composition in achieving reliable and reproducible results.

According to alternative embodiments (not shown), the detection elements 120 could be designed to bind different types of molecules, such as nucleic acids or small molecules, depending on the application.

A first example of immobilization molecules to be applied is given in the following:

Biomarker to be detected: Recombinant human IL-6 protein (R&D Systems 206-IL). Capture Anti-bodies: Human IL-6 MAb (Clone 973132) (R&D Systems MAB 9540). Detection Anti-bodies: Human IL-6 antibody (R&D Systems MAB2063).

A second example of immobilization molecules to be applied is given in the following:

Biomarker to be detected: Recombinant human TNF-alpha protein (R&D Systems 210-TA). Capture Anti-bodies: Human TNF-alpha antibody (R&D Systems MAB610). Detection Anti-bodies: Human TNF- alpha antibody (R&D Systems BAF210).

A third example of immobilization molecules to be applied is given in the following:

Biomarker to be detected: Recombinant human IL-8/CXCL8 protein (R&D Systems 208-IL). Capture Anti-bodies: Human IL-8/CXCL8 antibody (R&D Systems MAB2081). Detection Anti-bodies: Human IL-8/CXCL8 antibody (R&D Systems MAB2082).

A fourth example of immobilization molecules to be applied is given in the following:

Biomarker to be detected: Recombinant human C-reactive protein/CRP protein (R&D Systems 1707-CR). Capture Anti-bodies: Human C-reactive protein/CRP antibody (R&D Systems MAB17073). Detection Anti-bodies: Human C-reactive protein/CRP antibody (R&D Systems MAB17072).

A fifth example of immobilization molecules to be applied is given in the following:

Biomarker to be detected: Recombinant anti-cardiac troponin I (cTnI) (Hytest Finland Cat.# RC4T21). Capture Anti-bodies: Troponin I cardiac (cTnI), antibody (Hytest Finland 4T21/4T21cc, M18cc). Detection Anti-bodies: Troponin I cardiac (cTnI), antibody (Hytest Finland 4T21/4T21cc, 4C2cc).

A sixth example of immobilization molecules to be applied is given in the following:
Biomarker to be detected: NT-proBNP (HyTest, Finland 8NT2). Capture Anti-bodies: Anti-NT-proBNP (HyTest, Finland 4NT1cc 15C4cc). Detection Anti-bodies: Anti-NT-proBNP (HyTest, Finland 4NT1cc 13G12cc).

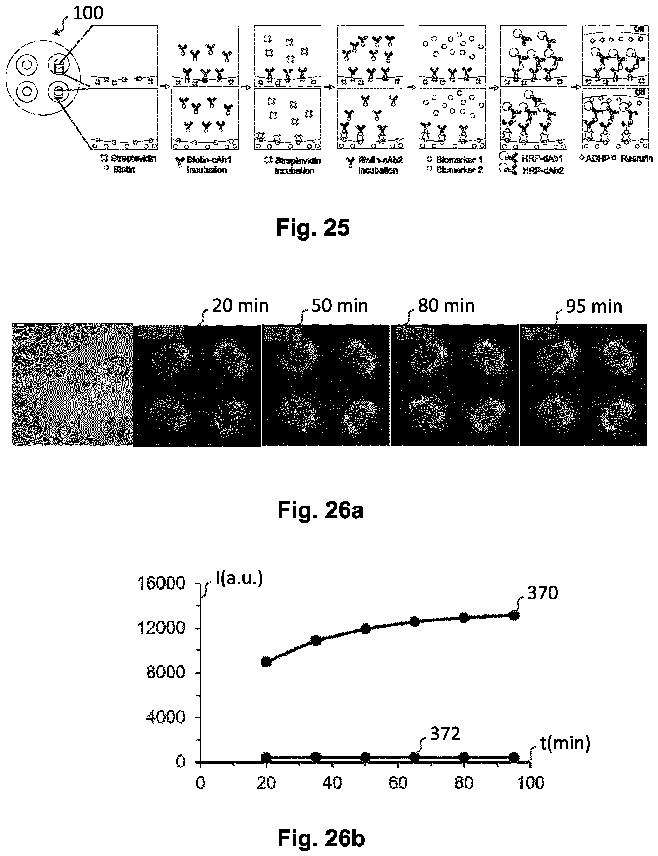

Fig. 25 shows an example of the microparticle 100, illustrating the process of immobilizing analytes from a fluid. The figure includes various stages of the process, each indicated by different symbols and annotations. The components shown in this figure include the microparticle 100, streptavidin, biotin, biotinylated antibodies (Ab1 and Ab2), biomarkers (Biomarker 1 and Biomarker 2), horseradish peroxidase HRP-conjugated antibodies (HRP-Ab1 and HRP-Ab2), and ADHP (10-Acetyl-3,7-dihydroxyphenoxazine) for the readout. Alternatively, 3,3',5,5'-Tetramethylbenzidine TMB can be applied. As a further alternative, a luminol substrate gives a fluorescence signal in the microscope that can be used in ELISA for readout, such as ADHP, TMB, β-galactosidase. Some of these are sensitive (like ADHP), while some are more stable (like β-galactosidase).

A microparticle 100 having detection elements 120 with molecule compositions comprising immobilization molecules of different types can be achieved by including acrylate-PEG-biotin in the first additional fluid (as described above in the context of the method 200), including acrylate-PEG- streptavidin in the second additional fluid (as described above in the context of the method 200). The preparation process of acrylate-PEG-streptavidin is demonstrated in Langmuir 2015, 31, 48,13165-13171. This document is herewith incorporated herein in its entirety by reference. Acrylate-PEG-streptavidin is prepared by mixing streptavidin and Acrylate-PEG-Succinimidyl Valerate (ACRL-PEG-SVA) at a mole ratio of 1:1. Streptavidin is chemically inert to ethanol and UV radiation, like biotin is, making it suitable for the method 200 described above.

The microparticle 100 comprises a body 110 with hydrophobic surfaces and detection elements 120 with hydrophilic surfaces. The detection elements 120 are shown to interact with different molecules during the incubation and binding processes. Streptavidin binds to biotin, which in turn binds to biotinylated antibodies (Ab1 and Ab2). These antibodies capture specific biomarkers (Biomarker 1 and Biomarker 2) from the fluid. HRP-conjugated antibodies (HRP-Ab1 and HRP-Ab2) then bind to these biomarkers, and the presence of HRP is detected using TMB, which produces an optical signal.

According to an embodiment, the process steps of Fig. 25 are similar to the process steps of Fig. 24, however, with an additional step of adding capture antibody-1 after particle fabrication. This results in a microparticle 100 having detection elements 120 with different molecule compositions at the respective surfaces, wherein the different molecule compositions comprise different immobilization molecules for capturing analytes of different types.

Mixing PEG linker (ACRL-PEG-SVA, Lysan Bio) with antibody results in ACR-PEG-Ab, EDC/NHS is a well-reported linking mechanism allowing acrylate linker to attach to antibody. This ACR-PEG-Ab is then cross-linked with hydrophilic PEGDA during the photo-polymerization of the stop-flow lithography process, making the hydrophilic layer functionalized with antibody. For further details, reference is made to Anal. Chem. 2011, 83, 1, 193-199 and to Nature Protocols volume 6, pages1761-1774 (2011). Both documents are herewith incorporated herein in their entirety by reference.

In a modification of the method of Fig. 25, nanoparticles coated with different antibodies are comprised in the first additional fluid and the second additional fluid (as described above in the context of the method 200), such that the resulting first and second detection element 120 of the same microparticle 100 fabricated comprise the nanoparticles coated with the different antibodies. The application of coated nanoparticles during photopolymerization has, for example been reported in Lab Chip, 2010,10, 3335-3340. This document is herewith incorporated herein in its entirety by reference.

Moreover, hydrogel droplet microarrays with trapped antibody-functionalized beads have been demonstrated in Lab Chip, 2011,11, 528- 534. This document is herewith incorporated herein in its entirety by reference.

Fig. 26a shows an example of the microparticle 100 during the incubation process, illustrating the time-dependent binding of analytes. The figure includes images taken at different time points (20 min, 60 min, and 100 min) to show the progression of the binding process.

The microparticle 100 is shown to have a body with multiple detection elements 120, half of them serving as detector, the other ones (not equipped with the molecule composition with immobilization molecule) serving as a reference. The detection elements 120 comprise cavities 122 which get filled with the surrounding fluid containing the analytes, and the binding process is monitored over time. The images show the increasing intensity of the signal as more analytes are captured by the detection elements.

Fig. 26b shows the quantitative analysis of the binding process illustrated in Fig. 26a, by giving the time-dependent increase in signal intensity. The figure includes a graph with time t on the x-axis and signal intensity on the y-axis, showing the increase in signal over time for (1) the cavities with the molecule composition with the immobilization molecules (upper data points 370) and (2) the reference cavities without the molecule composition with the immobilization molecules (lower data points 372). The data points represent the mean signal intensity, and the error bars represent the standard deviation, indicating the variability of the measurements.

The graph shows that the signal intensity increases over time as more analytes are captured by the detection elements 120.

The advantage of quantitative analysis is that it provides a precise measurement of the amount of analyte captured by the detection elements 120, allowing for accurate quantification of the analytes in the fluid sample. This is particularly important for applications where the concentration of the analyte is critical, such as in clinical diagnostics or environmental monitoring.

By monitoring the intensity of signal under the microscope, the concentration of the analyte can be estimated.

The examples of the present disclosure disclosed herein only constitute specific examples for illustration purposes. The present invention can be implemented in various ways and with many modifications without altering the underlying basic properties. Therefore, the present invention is only defined by the claims as stated below.

## Claims

1. A microparticle (100) for immobilizing analytes from a fluid, the microparticle (100) comprising:
a body (110) comprising a hydrophobic surface; and
detection elements (120), the detection elements (120) comprising a first detection element comprising a first hydrophilic surface and a second detection element comprising a second hydrophilic surface;
wherein the detection elements (120) are mechanically interconnected by means of the body (110);
wherein the first detection element comprises, at the first hydrophilic surface, a first molecule composition for immobilizing analytes from the fluid at the first hydrophilic surface, and the second detection element comprises, at the second hydrophilic surface, a second molecule composition for immobilizing analytes from the fluid at the second hydrophilic surface;
wherein the first molecule composition is different from the second molecule composition; and
wherein the microparticle (100) is non-mirror symmetric about any plane which does not intersect all of the detection elements (120).

2. The microparticle (100) according to claim 1, wherein the body (110) comprises a marker structure (112), wherein the marker structure (112) is non-mirror symmetric about any plane which does not intersect all of the detection elements (120).

3. The microparticle (100) according to claim 2, wherein the marker structure (112) is associated with an outer surface of the body (110), in particular, wherein the marker structure (112) is formed by the outer surface of the body (110) or is arranged on the outer surface of the body (110);
wherein, optionally, the body (110) has a top surface (114, 116) and a bottom surface (114, 116) opposite to the top surface (114, 116), wherein at least part of the marker structure (112) is associated with the top surface (114, 116) and/or with the bottom surface (114, 116),
in particular, wherein the at least part of the marker structure (112) is associated with a circumferential shape of the top surface (114, 116) and/or with a circumferential shape of the bottom surface (114, 116),
in particular, wherein the marker structure (112) comprises recesses in the circumferential shape of the top surface (114, 116) and/or in the circumferential shape of the bottom surface (114, 116),
in particular, wherein at least two of said recesses have different shapes and/or at least two of said recesses are comprised in the same one of the circumferential shape of the top surface (114, 116) and the circumferential shape of the bottom surface (114, 116),
in particular, wherein said at least two of the recesses have different shapes and are both comprised in both the circumferential shape of the top surface (114, 116) and the circumferential shape of the bottom surface (114, 116).

4. The microparticle (100) according to any of the preceding claims, comprising at least two different marker materials having different optical properties, wherein the at least two different marker materials are arranged in the microparticle (100) non-mirror symmetrically about any plane which does not intersect all of the detection elements (120);
wherein, optionally:
the first detection element comprises a first marker material of the at least two different marker materials at a higher concentration, in particular in terms of weight percent or in terms of molar concentration, than the second detection element, and the second detection element comprises a second marker material of the at least two different marker materials at a higher concentration, in particular in terms of weight percent or in terms of molar concentration, than the first detection element, in particular, wherein said higher concentrations are higher at least by a factor of two or at least by a factor of five or at least by a factor of ten;
wherein, optionally:
the first detection element comprises a first marker material of the at least two different marker materials at a higher concentration, in particular in terms of weight percent or in terms of molar concentration, than the body (110), in particular, at least by a factor of two or at least by a factor of five or at least by a factor of ten; and/or
the second detection element comprises a second marker material of the at least two different marker materials at a higher concentration, in particular in terms of weight percent or in terms of molar concentration, than the body (110), in particular, at least by a factor of two or at least by a factor of five or at least by a factor of ten;
and/or
wherein the different optical properties comprise at least one of the following: different photoluminescence emission wavelengths, and maxima at different wavelengths in corresponding optical absorption spectra; and/or
wherein the at least two different marker materials comprise or are at least two different photoluminescent dyes, in particular, wherein the at least two different marker materials comprise or are at least two different fluorescent dyes.

5. The microparticle (100) according to any of the preceding claims, wherein the analytes comprise analytes of a first type;
wherein the first molecule composition comprises immobilization molecules of a first type at a higher concentration than the second molecule composition, wherein the immobilization molecules of the first type are adapted to immobilize analytes of the first type at the respective surface comprising the respective molecule composition comprising the immobilization molecules of the first type;
wherein optionally:
the immobilization molecules of the first type are adapted to selectively immobilize the analytes of the first type at the respective surface comprising the respective molecule composition comprising the immobilization molecules of the first type; and/or
the higher concentration refers to a higher concentration in terms of weight percent or in terms of molar concentration; and/or
the higher concentration is higher at least by a factor of two, or at least by a factor of five, or at least by a factor of ten; and/or
the immobilization molecules of the first type are essentially absent in the second molecule composition.

6. The microparticle (100) according to any of the preceding claims, wherein the analytes comprise analytes of a first type and analytes of a second type;
wherein the first molecule composition comprises immobilization molecules of a first type for selectively immobilizing the analytes of the first type from the fluid at the first hydrophilic surface; and
wherein the second molecule composition comprises immobilization molecules of a second type for selectively immobilizing the analytes of the second type from the fluid at the second hydrophilic surface;
wherein, optionally:
the microparticle (100) for immobilizing the analytes from the fluid refers to a microparticle (100) for immobilizing analytes of the first type and of the second type different from the first type from the fluid, in particular, selectively at different surface regions along the microparticle (100); and/or
the first molecule composition comprises the immobilization molecules of the first type at a higher concentration, in particular in terms of weight percent or in terms of molar concentration, than the second molecule composition and optionally than the body (110), in particular, at least by a factor of two or at least by a factor of five or at least by a factor of ten; and/or
the second molecule composition comprises the immobilization molecules of the second type at a higher concentration, in particular in terms of weight percent or in terms of molar concentration, than the first molecule composition and optionally than the body (110), in particular, at least by a factor of two or at least by a factor of five or at least by a factor of ten.

7. The microparticle (100) according to any of the preceding claims,
wherein the first detection element comprises a first cavity (122), and at least part of the first hydrophilic surface is a surface of the first cavity (122), and the second detection element comprises a second cavity (122), and at least part of the second hydrophilic surface is a surface of the second cavity (122);
wherein, optionally:
the first cavity (122) and the second cavity (122) are separated from one another by means of the body (110); and/or
said surface of the first cavity (122) refers to an inner surface of the first cavity (122) and said surface of the second cavity (122) refers to an inner surface of the second cavity (122); and/or
the first cavity (122) and/or the second cavity (122) is formed as a through hole extending from a bottom surface (114, 116) of the microparticle (100) to a top surface (114, 116) of the microparticle (100), the top surface (114, 116) and the bottom surface (114, 116) being opposite surfaces of the microparticle (100);
and/or
wherein the detection elements (120) are embedded in the body (110), in particular, wherein the first cavity (122) and the second cavity (122) are embedded in the body (110).

8. The microparticle (100) according to any of the preceding claims,
wherein the microparticle (100) has a thickness (t) of less than 1 mm,
in particular from a top surface (114, 116) of the microparticle (100) to a bottom surface (114, 116) of the microparticle (100), the bottom surface (114, 116) being located opposite to the top surface (114, 116),
in particular, wherein the top surface (114, 116) and the bottom surface (114, 116) are parallel to each other and/or wherein an aspect ratio between the thickness (t) of the microparticle (100) and a width (w) of the top surface (114, 116) and/or of the bottom surface (114, 116) is no more than 1:1.5 or no more than 1:2 or no more than 1:2.5 or no more than 1:3; and/or
wherein the microparticle (100) has a width (w) of less than 3 mm, in particular of less than 1 mm and/or along any spatial direction; and/or
wherein the microparticle (100) is shaped to permit, when the microparticle (100) is placed in the fluid, the fluid to reach the first hydrophilic surface and to reach the second hydrophilic surface; and/or
wherein the body (110) comprises or consists of a body material, the body material being hydrophobic; and/or
wherein the first detection element comprises or consists of a first material, the first material being hydrophilic; and/or
wherein the second detection element comprises or consists of a second material, the second material being hydrophilic, in particular, wherein the second material is the same as the first material; and/or
wherein the first detection element and the second detection element are encircled by a top surface (114, 116) of the microparticle (100), in particular, wherein the first detection element and the second detection element are encircled by the top surface (114, 116) of the microparticle (100) and by a bottom surface (114, 116) of the microparticle (100), the top surface (114, 116) and the bottom surface (114, 116) being opposite surfaces (114, 116) of the microparticle (100); and/or
wherein the detection elements (120) further comprise a third detection element comprising a third hydrophilic surface, wherein the third detection element at the third hydrophilic surface comprises a third molecule composition for immobilizing analytes from the fluid at the third hydrophilic surface, wherein the third molecule composition is different from the first molecule composition and from the second molecule composition;
in particular, wherein the detection elements (120) further comprise a fourth detection element comprising a fourth hydrophilic surface, wherein the fourth detection element at the fourth hydrophilic surface comprises a fourth molecule composition for immobilizing analytes from the fluid at the fourth hydrophilic surface, wherein the fourth molecule composition is different from the first molecule composition, from the second molecule composition and from the third molecule composition; and/or
the detection elements (120) are separated from one another by means of the body (110).

9. A plurality of microparticles (100) according to any of the preceding claims,
wherein each microparticle (100) of the plurality of microparticles (100) comprises the marker structure (112) according to claim 2 or 3 and/or comprises the at least two different marker materials according to claim 4;
in particular, with a same arrangement thereof in the microparticles (100) of the plurality of microparticles (100).

10. A method (200) for fabricating the microparticle (100) according to any of the preceding claims, the method (200) comprising:
providing (210) parallel flows of fluids in a flow channel (302), said parallel flows of fluids comprising:
a flow of a fluid comprising a precursor for a hydrophobic material;
a flow of a first additional fluid, the first additional fluid comprising a precursor for a hydrophilic material; and
a flow of a second additional fluid, the second additional fluid comprising a precursor for a hydrophilic material;
curing (220) the precursor for the hydrophobic material into a hydrophobic material forming at least part of the body (110);
curing (230) the precursor for the hydrophilic material comprised in the first additional fluid into a hydrophilic material forming at least part of the first detection element; and
curing (240) the precursor for the hydrophilic material comprised in the second additional fluid into a hydrophilic material forming at least part of the second detection element;
wherein, optionally, the provided parallel flows of the fluids comprise a common solvent and/or the first additional fluid and the second additional fluid comprise immobilization molecules, such that the first molecule composition comprises immobilization molecules from the first additional fluid and the second molecule composition comprises immobilization molecules from the second additional fluid, in particular, wherein the immobilization molecules comprised in the first additional fluid comprise immobilization molecules of a first type and the immobilization molecules comprised in the second additional fluid comprise immobilization molecules of a second type.

11. An apparatus (300) for fabricating the microparticle (100) according to any of claims 1 to 8, the apparatus (300) comprising a flow channel (302), the flow channel (302) comprising:
at least three inlet openings (310) for providing (210) parallel flows of fluids in the flow channel (302), wherein each of the at least three inlet openings (310) is adapted to provide a respective one of the parallel flows of the fluids in the flow channel (302);
a reducing section (320) downstream from the at least three inlet openings (310), wherein a cross-sectional area of the flow channel (302) at a downstream end of the reducing section (320) is smaller than a cross-sectional area of the flow channel (302) at an upstream end of the reducing section (320);
tapered elements (330) arranged in the flow channel (302) and at least partially in the reducing section (320) of the flow channel (302), each of the tapered elements (330) having a smaller cross-sectional area at a downstream end of the respective tapered element than at an upstream end of the respective tapered element;
wherein the at least three inlet openings (310) comprise a first inlet opening (312) located in the flow channel (302) at a first lateral position and a second inlet opening (314) located in the flow channel (302) at a second lateral position laterally offset from the first lateral position; and
wherein the tapered elements (330) comprise:
a first tapered element (332) arranged downstream from the first inlet opening (312) at a lateral position corresponding to the first lateral position, to reduce a cross-sectional area of the one of the parallel flows of the fluids provided by the first inlet opening (312), and
a second tapered element (334) arranged downstream from the second inlet opening (314) at a lateral position corresponding to the second lateral position, to reduce a cross-sectional area of the one of the parallel flows of the fluids provided by the second inlet opening (314).

12. The apparatus (300) according to claim 11, wherein the tapered elements (330) further comprise an outer tapered element (336) around the first tapered element (332) and the second tapered element (334),
wherein, optionally:
the outer tapered element (336) surrounds the first tapered element (332) and the second tapered element (334) in a plane perpendicular to a flow direction defined by the flow channel (302); and/or
the outer tapered element (336) has an asymmetric shape around a centerline of the flow channel (302), in particular, wherein the asymmetric shape refers to a shape without a rotational symmetry around the center line, in particular, to a shape without a discrete or continuous rotational symmetry around the centerline; and/or
the outer tapered element (336) comprises at least one notch, in particular, at least two notches at different lateral positions along the outer tapered element (336), in particular, wherein the at least two notches at the different lateral positions along the outer tapered element (336) have different sizes and/or the at least two notches further comprise a third notch.

13. The apparatus (300) according to claim 11 or 12,
wherein the at least three inlet openings (310) comprise at least one outer inlet opening (316, 318); and
wherein, according to a projection onto a plane perpendicular to a flow direction defined by the flow channel (302), each of the at least one outer inlet opening (316, 318) is arranged around the first inlet opening (312) and around the second inlet opening (314);
in particular, wherein the at least one outer inlet opening (316, 318) comprises a first outer inlet opening (316),
in particular, wherein the outer tapered element (336) of claim 12 is arranged downstream from the first outer inlet opening (316) and, according to the projection onto the plane perpendicular to the flow direction defined by the flow channel (302): the upstream end of the outer tapered element (336) is arranged around the first outer inlet opening (316) and/or the outer tapered element (336) overlaps the first outer inlet opening (316);
in particular, wherein the at least one outer inlet opening (316, 318) comprises a second outer inlet opening (318);
in particular, wherein the outer tapered element (336) of claim 12 is arranged downstream from the second outer inlet opening (318) and, according to the projection onto the plane perpendicular to the flow direction defined by the flow channel (302): the second outer inlet opening (318) is arranged around the downstream end of the outer tapered element (336) and/or the outer tapered element (336) overlaps the second outer inlet opening (318).

14. The apparatus (300) according to any of claim 11 to 13,
wherein the apparatus (300) further comprises a lithographic mask (352) arranged in a lithographic masking section (350) of the apparatus (300), wherein a section (302m) of the flow channel (302) passes through said lithographic masking section (350), and wherein the lithographic mask (352) is arranged in the lithographic masking section (350) such that, under illumination of the mask with a UV radiation (354) having a direction towards said section (302m) of the flow channel (302), said UV radiation (354) is masked with the lithographic mask (352) before reaching said section (302m) of the flow channel (302);
in particular:
wherein the lithographic mask (352) comprises slits (20) having segments (20a) with an extension perpendicular to a flow direction defined by the flow channel (302) in the lithographic masking section (350), said slits (20) being adapted to allow said UV radiation (354) to reach said section (302m) of the channel, wherein the lithographic mask (352) is adapted to block at least a part of the UV radiation (354) or the UV radiation (354) not passing through the slits (20); and/or
wherein the apparatus (300) further comprises a UV source (356) adapted to provide said UV radiation (354) having the direction towards said section (302m) of the flow channel (302).

15. The method (200) according to claim 10,
wherein the method (200) applies the apparatus (300) of any of claims 11 to 14;
wherein the parallel flows of the fluids in the flow channel (302) are provided via the at least three inlet openings (310);
wherein the flow of the first additional fluid is provided from the first inlet opening (312); and
wherein the flow of the second additional fluid is provided from the second inlet opening (314);
wherein, optionally:
the flow of the fluid comprising the precursor for the hydrophobic material is provided from one of the at least one outer inlet opening (316, 318) of claim 13, in particular, from the first outer inlet opening (316) of claim 13; and/or
the parallel flows of the fluids provided in the flow channel (302) further comprise a carrier flow, in particular, wherein the carrier flow is provided from one of the at least one outer inlet opening (316, 318) of claim 13, in particular, from the second outer inlet opening (316) of claim 13.
